(19) Europäisches Patentamt
European Patent Office
Office européen des brevets

(11) **EP 3 201 359 B1**

(12) **EUROPEAN PATENT SPECIFICATION**

(45) Date of publication and mention
of the grant of the patent:
**04.12.2019 Bulletin 2019/49**

(51) Int Cl.:
**C12Q 1/6851** (2018.01)   **C12Q 1/686** (2018.01)
**G16B 40/00** (2019.01)

(21) Application number: **15847004.7**

(22) Date of filing: **30.09.2015**

(86) International application number:
**PCT/KR2015/010308**

(87) International publication number:
**WO 2016/052991 (07.04.2016 Gazette 2016/14)**

(54) **METHODS FOR ANALYZING SAMPLES**

VERFAHREN ZUR ANALYSE VON PROBEN

PROCÉDÉS D'ANALYSE D'ÉCHANTILLONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.10.2014 KR 20140132229
29.12.2014 KR 20140191924
10.04.2015 KR 20150051080**

(43) Date of publication of application:
**09.08.2017 Bulletin 2017/32**

(73) Proprietor: **Seegene, Inc.
Seoul 05548 (KR)**

(72) Inventors:
• **CHUN, Jong Yoon
Seoul 06075 (KR)**
• **LEE, Young Jo
Seoul 05507 (KR)**

(74) Representative: **advotec.
Patent- und Rechtsanwälte
Widenmayerstrasse 4
80538 München (DE)**

(56) References cited:
**EP-A2- 1 801 237      EP-A2- 1 804 172
WO-A2-2011/130184      US-A1- 2007 111 249
US-A1- 2007 248 982      US-A1- 2012 231 458
US-B2- 7 363 168      US-B2- 7 720 611**

• **RUIJTER ET AL.: 'Amplification efficiency:
linking baseline and bias in the analysis of
quantitative PCR data' NUCLEIC ACIDS
RESEARCH vol. 37, no. 6, 01 April 2009, pages 1
- 12, XP055424526 DOI: 10.1093/NAR/GKP045**

<110> Seegene, Inc.

<120> Methods for Analyzing Samples

<130> PP150073

<150> KR 10-2014-0132229
<151> 2014-10-01

<150> KR 10-2014-0191924
<151> 2014-12-29

<150> KR 10-2015-0051080
<151> 2015-04-10

<160> 6

<170> KoPatent in 3.0

<210> 1
<211> 37
<212> DNA
<213> Artificial Sequence

<220>
<223> NG-F

<220>
<221> misc_feature
<222> (22)..(26)
<223> n denotes deoxyinosine

<400> 1
tacgcctgct actttcacgc tnnnnngtaa tcagatg        37

<210> 2
<211> 37
<212> DNA
<213> Artificial Sequence

<220>
<223> NG-R

<220>
<221> misc_feature
<222> (22)..(26)
<223> n denotes deoxyinosine

<400> 2
caatggatcg gtatcactcg cnnnnncgag caagaac        37

<210> 3
<211> 22
<212> DNA
<213> Artificial Sequence

<220>
<223> NG-P

<400> 3
tgcccctcat tggcgtgttt cg        22

<210> 4
<211> 37

<212> DNA
<213> Artificial Sequence

<220>
<223> Flu A-F

<220>
<221> misc_feature
<222> (23)..(27)
<223> n denotes deoxyinosine

<400> 4
tggaatggct aaagacaaga ccnnnnntgt cacctct        37

<210> 5
<211> 36
<212> DNA
<213> Artificial Sequence

<220>
<223> Flu A-R

<220>
<221> misc_feature
<222> (25)..(28)
<223> n denotes deoxyinosine

<400> 5
catcctgttg tatatgaggc ccatnnnnct ggcaag        36

<210> 6
<211> 23
<212> DNA
<213> Artificial Sequence

<220>
<223> Flu A-P

<400> 6
ctcactgggc acggtgagcg tga        23

**Description**

**BACKGROUND OF THE INVENTION**

**FIELD OF THE INVENTION**

The present invention relates to a method for analyzing a sample. In particular, the present invention relates to a method for analyzing a sample and a method for correcting a raw data set of an amplification reaction.

**DESCRIPTION OF THE RELATED ART**

Analyzing samples are critical in various fields of technologies. The analyses of samples are conducted for elucidating, describing or characterizing samples in view of certain properties.

In the biotechnological field, the analyses of samples have much more importance. Particularly, the analyses of samples are generally performed to provide information as to certain characteristics including the presence or absence of analytes, binding affinity, enzyme activity, gene expression levels and amino acid or nucleotide sequences. As representatives, an immunoassay and genetic analysis have been widely conducted to analyze samples. There have been published patents for analyzing biosamples such as U.S. Pat. Nos. 6516276, 6228593, 7349809, 7115229 and 6816790.

A target nucleic acid amplification process is prevalently involved in most of technologies for detecting target nucleic acid molecules. Nucleic acid amplification is a pivotal process for a wide variety of methods in molecular biology, such that various amplification methods have been proposed. The most predominant process for nucleic acid amplification known as polymerase chain reaction (hereinafter referred to as "PCR") is based on repeated cycles of denaturation of double-stranded DNA, followed by oligonucleotide primer annealing to the DNA template, and primer extension by a DNA polymerase (Mullis et al. U.S. Pat. Nos. 4,683,195, 4,683,202, and 4,800,159; Saiki et al., (1985) Science 230, 1350-1354).

A real-time PCR is one of PCR-based technologies for detecting a target nucleic acid molecule in a sample in a real-time manner (Logan J et al., (2009). Real-Time PCR: Current Technology and Applications. Caister Academic Press). For detecting a target nucleic acid molecule, the real-time PCR uses a signal-generating means for generating a fluorescent signal being detectable in a proportional manner with the amount of the target molecule. The generation of fluorescent signals may be accomplished by using either intercalators generating signals when intercalated between double-stranded DNA or oligonucleotides carrying fluorescent reporter and quencher molecules. The fluorescent signals whose intensities are proportional with the amount of the target molecule are detected at each amplification cycle and plotted against amplification cycles, thereby obtaining an amplification curve or amplification profile curve.

In general, an amplification curve of the real-time PCR may be classified into a baseline region, an exponential phase, linear phase and a plateau phase. The exponential phase shows increase in fluorescent signals in proportional to increase of amplification products. In the linear phase, the increase in fluorescent signals is substantially reduced and behaves in a substantially linear manner and the plateau phase refers to a region in which there is little increase in fluorescent signals due to saturation of both PCR amplicon and fluorescent signal levels.

The baseline region refers to a region in which there is little change in fluorescent signal during initial cycle of PCR. In the baseline region, the level of PCR amplicon is not sufficient to be detectable and therefore signals detected in this region may be due to background signal involving fluorescent signals from reaction reagents and measurement device.

For analyzing data of the real-time PCR in more accurate and reproducible manner, the correction (or normalization) of an amplification curve has to be made. The amplification curve may be corrected by determination of a baseline region and removal of a background signal in the baseline region.

As the background signal reflects change in reaction conditions and environments of PCR, the background signal is very likely to be differently generated for each PCR reaction and therefore a baseline drift is often observed irrespective of the amount of a target nucleic acid molecule. The baseline drift makes it difficult to compare amplification curves of different PCR reactions and may contribute to false-positive or false-negative detection results. Therefore, in analysis of PCR data, there is needed in establishment of a suitable baseline region and correction of experimental data of PCR based on the established baseline region.

As conventional approaches for correction of amplification curves, an arbitrarily determined cycle region during initial cycles of PCR (*e.g.*, 3-15 cycles) has been determined as a baseline region. Another approach includes obtaining experimentally an amplification curve and then establishing a baseline region with determining a cycle before an amplification signal significantly increases. U.S. Pat. No. 8,219,324 discloses that a second derivative of an amplification curve is calculated and a baseline region is established with a data point having certain characteristics as an end-point cycle.

The conventional approaches have some serious drawbacks.

In the above-described method in which a baseline is arbitrarily pre-determined with an initial cycle region, the method does not correct a baseline drift while it may correct change in background signals being different for each PCR reaction. The baseline region pre-determined cannot be applied to various samples because a start-point of an exponential region varies depending on an initial level of a target molecule in a sample. In the above-described method in which a baseline region is arbitrarily determined by a researcher, baseline regions for the same amplification curve are likely to be different depending on researchers to analyze, which leads to no reproducible analysis results.

The technologies taught by U.S. Pat. No. 8,219,324 using complicated algorithms for determining a baseline region require a number of parameters not well-defined in the algorithms of which optimization may become troublesome.

In various sample analysis methods using threshold values, the occurrence of noise signals or non-typical patterned signals (e.g., negative slope pattern) is very likely to result in false positive or false negative results. Such analysis errors would be hard to be removed by methods using conventional threshold values. Several publications deal with the use of threshold values or baselines and computer implemented methods for using or correcting them (US2012/231458, WO2011/130184, EP1804172, EP1801237).

Accordingly, there are strong needs in the art to develop novel approaches for improving the sample analysis method (e.g., correcting an amplification curve) by establishing threshold in new approaches or a more-accurate baseline region for each sample (or PCR reaction), which contributes to more accurate and reliable analysis results.

## SUMMARY OF THE INVENTION

The present inventors have made intensive researches to develop novel approaches for obtaining more accurate and reliable results of a signal-generating process by processing a data set obtained from the signal-generating process, thereby providing analysis results of a sample in a more accurate and reliable manner. As results, we have found that a variable threshold of which the threshold values for at least two cycles among cycles are different from each other is applied to cycles of a signal-generating process for effectively eliminating hindrance factors in determining significance of signals from the signal-generating process or incorrect signals not representing a true increase in signals from the signal-generating process. The present invention has been found to be excellently applied to correction of a raw data set of an amplification reaction.

Accordingly, it is an object of this invention to provide a method for analyzing a sample using a variable threshold.

It is another object of this invention to provide a method for correcting a raw data set of an amplification reaction using a signal-generating means.

It is still another object of this invention to provide a computer readable storage medium containing instructions to configure a processor to perform a method for analyzing a sample.

It is further object of this invention to provide a computer readable storage medium containing instructions to configure a processor to perform a method for correcting a raw data set of an amplification reaction using a signal-generating means.

It is still further object of this invention to provide a device for analyzing a sample.

It is another object of this invention to provide a device for correcting a raw data set of an amplification reaction using a signal-generating means.

It is still another object of this invention to provide a computer program to be stored on a computer readable storage medium to configure a processor to perform a method for analyzing a sample.

It is further object of this invention to provide a computer program to be stored on a computer readable storage medium to configure a processor to perform a method for correcting a raw data set of an amplification reaction using a signal-generating means.

Other objects and advantages of the present invention will become apparent from the detailed description to follow taken in conjugation with the appended claims and drawings.

## BRIEF DESCRIPTION OF THE DRAWINGS

Fig. 1a represents a flow diagram illustrating an embodiment of the present method for analyzing a sample.

Fig. 1b represents a flow diagram illustrating an embodiment of the present method for correcting an amplification curve.

Fig. 2 represents a pre-corrected amplification showing results of the real-time PCR over 50 cycles using Taqman probe as a signal-generating means. The real-time PCR was performed in the presence of a signal-generating means to obtain a raw data set containing amplification cycle numbers and measured signals. The raw data set was plotted. RFU denotes relative fluorescence unit.

Fig. 3 represents a curve of slopes for the pre-corrected amplification curve of Fig. 2. Y-axis represents a slope calculated for each of the amplification cycles using the raw data set. The slopes were calculated by a least square method. S is the start-point cycle and E is the end-point cycle of the baseline region, wherein one of the early cycles was determined as a start-point cycle(S) and the first cross-point cycle between the baseline threshold and the slope curve was determined as an end-point cycle(E).

Fig. 4a shows a magnification of a baseline region of the pre-corrected amplification curve of Fig. 2 (raw data set) and a best-fit line (linear regression line) depicted by a function for a best-fit obtained by a least square method. S is the start-point cycle and E is the end-point cycle of the baseline region.

Fig. 4b shows a magnification of a baseline region of a corrected amplification curve by subtracting values of the function for the best-fit line from the values of the measured signals of the raw data set. S is the start-point cycle and E is the end-point cycle of the baseline region.

Fig. 5 shows procedures for obtaining a corrected amplification curve of Fig. 2 in which fluorescent signal intensities of the raw data set of Fig. 2 were subtracted by values of the function for the best-fit line (linear regression line) to obtain a corrected data set, followed by plotting the corrected data set.

Fig. 6 schematically represents an embodiment of a real-time PCR system equipped with a program for analyzing samples by the present invention.

Fig. 7 shows that using a fixed baseline threshold value ("300" or "30") over all amplification cycles for determining an end-point cycle of a baseline region may result in erroneous establishment of the baseline region.

Figs. 8a and 8b represent embodiments for establishment of a baseline region for high-concentrated and low-concentrated samples, respectively. The end-point cycle was determined as a cycle after a minimum baseline end-point cycle (MBEC) among cross-point cycles between the slope curve and the baseline threshold.

Fig. 8c represents results of correction of an amplification curve by using a baseline region established with or without the MBEC for determining an end-point cycle.

Fig. 9 schematically represents various embodiments in which baseline threshold values that may vary depending on cycles are applied to an amplification curve (or a slope curve). The bold lines depict baseline thresholds. BTCC depicts a baseline threshold-changed cycle.

Figs. 10a and 10b represent results of application of the VBT (Variable Baseline Threshold) to a slope curve for high-concentrated and low-concentrated samples, respectively. The end-point cycle was determined as a cross-point cycle between the slope curve and the baseline threshold values differently adopted with respect to a baseline threshold-changed cycle (BTCC).

Fig. 10c represents results of correction of an amplification curve by using a baseline region established with or without the VBT for determining an end-point cycle.

Fig. 11a represents results of application of the VST (Variable Signal Threshold) to an amplification curve for determination of Ct values. FST (fixed signal threshold) method refers to a conventional technology.

Figs. 11b and 11c represent results of application of the VST (Variable Signal Threshold) to amplification curves of serially diluted genomic RNAs ($10^{-3}$, $10^{-4}$, $10^{-5}$, $10^{-6}$, and $10^{-7}$ dilutions) of Flu A for determination of $C_t$ values

## DETAILED DESCRIPTION OF THIS INVENTION

In the Specification, descriptions for common technologies and knowledge well known in the art and directly unrelated to the present invention are omitted such that the Specification becomes more descriptive and explanatory for the present invention. Furthermore, the common descriptions between the Sections described below are omitted in order to avoid undue redundancy leading to the complexity of this Specification.

### I. Analyzing a sample using a variable threshold

In one aspect of this invention, there is provided a method for analyzing a sample, comprising:

(a) obtaining a value of signal at each of cycles of a signal-generating process using the sample to provide values of signals at the cycles;
(b) applying a threshold value to each of the cycles such that a plurality of threshold values are applied to the cycles; wherein the threshold values of at least two cycles among the cycles are different from each other;
(c) identifying one or more cycles satisfying a threshold criterion determined by each of the threshold values; and
(d) analyzing the sample by using the identified cycle or cycles in the step (c).

The present invention is directed to analyze a sample by using and processing a data set (*e.g.,* values of signals and cycles) from a signal-generating process.

According to an embodiment of this invention, the analyzing the sample is to determine the presence or absence of an analyte in the sample. The term used "determining the presence or absence of an analyte in a sample" means determining qualitatively or quantitatively the presence or absence of an analyte in a sample.

The present inventors have made intensive researches to develop novel approaches for obtaining more accurate and reliable results of a signal-generating process by processing a data set obtained from the signal-generating process, thereby providing analysis results of a sample in a more accurate and reliable manner. As results, we have found that a variable threshold of which the threshold values for at least two cycles among cycles are different from each other is applied to cycles of a signal-generating process for effectively eliminating hindrance factors in determining significance of signals from the signal-generating process or incorrect signals not representing a true increase in signals from the signal-generating process. The present invention has been found to be excellently applied to correction of a raw data set of an amplification reaction.

To our best knowledge, there has not been yet reported our approach that a threshold values of at least two cycles among the cycles have different threshold values from each other are applied to cycles of a signal-generating process.

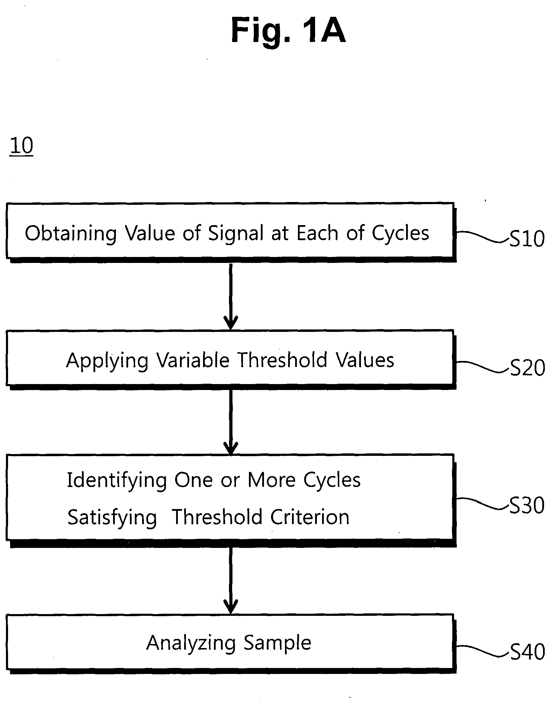

Fig. 1a represents a flow diagram illustrating an embodiment of the present method for analyzing a sample. The present invention will be described in more detail as follows:

## Step (a): Obtaining Values of Signals(S10)

First, a value of signal at each of cycles of a signal-generating process using the sample is obtained to provide values of signals at the cycles.

The present invention is directed to analyze a sample by using and processing a data set *(e.g.,* values of signals and cycles) from a signal-generating process. The step (a) may be also described as obtaining a data set containing (i) cycles of a signal-generating process using the sample and (ii) values of signals of the signal-generating process at the cycles.

The term used herein "signal-generating process" refers to any process capable of generating signals in a dependent manner on the presence of an analyte in a sample.

The signal-generating process is accompanied with signal change.

According to an embodiment, the signal-generating process is a signal amplification process.

The term "signal" as used herein refers to a measurable output.

The signal change may serve as an indicator indicating qualitatively or quantitatively the presence or absence of an analyte.

Examples of useful indicators include fluorescence intensity, luminescence intensity, chemiluminescence intensity, bioluminescence intensity, phosphorescence intensity, charge transfer, voltage, current, power, energy, temperature, viscosity, light scatter, radioactive intensity, reflectivity, transmittance and absorbance. The most widely used indicator is fluorescence intensity.

According to an embodiment, the signal-generating process is a process to provide an amplification curve. Particularly, the amplification curve is a signal amplification curve.

Such signal-generating process may include biological and chemical processes. The biological processes may include genetic analysis processes such as PCR, real-time PCR, microarray and invader assay, immunoassay processes and bacteria growth analysis. Particularly, the signal-generating process includes genetic analysis processes. Chemical process may include chemical analysis comprising production, change or decomposition of chemical materials.

The analyte may include biological materials such as nucleic acid molecules *(e.g.,* DNA and RNA), proteins, peptides, carbohydrates, lipids, amino acids, biological chemicals, hormones, antibodies, antigens, metabolites and cells. Alternatively, the analyte may include non-biological materials such as chemicals.

According to an embodiment of this invention, the analyte is a target nucleic acid molecule. The term "target nucleic acid molecule" means a nucleic acid molecule to be detected or analyzed.

According to an embodiment of this invention, the signal-generating process is a process with amplification or with no amplification of an analyte.

Particularly, the signal-generating process is a process with amplification of an analyte, more particularly, a target nucleic acid molecule. Much more particularly, the signal-generating process is a process with amplification of a target nucleic acid molecule and capable of increasing or decreasing signals (particularly, increasing signals) upon amplifying the target nucleic acid molecule.

The term used herein "signal generation" include appearance or disappearance of signals and increase or decrease in signals. Particularly, the term "signal generation" means increase in signals.

According to an embodiment of this invention, the signal-generating process is performed in the presence of a signal-generating means.

The term used herein "signal-generating means" refers to any material used in generation of signals indicating the presence of the analyte (e.g., target nucleic acid molecules), for example including oligonucleotides, labels and enzymes. Alternatively, the term used herein "signal-generating means" can be used to refer to any methods using the materials for signal generation.

A wide variety of the signal-generating means have been known to one of skill in the art. The signal-generating means include both labels *per se* and oligonucleotides with labels. The labels may include a fluorescent label, a luminescent label, a chemiluminescent label, an electrochemical label and a metal label. The label *per se* like an intercalating dye may serve as signal-generating means. Alternatively, a single label or an interactive dual label containing a donor molecule and an acceptor molecule may be used as signal-generating means in the form of linkage to at least one oligonucleotide.

The signal-generating means may comprise additional components for generating signals such as nucleolytic enzymes (e.g., 5'-nucleases and 3'-nucleases).

Where the present method is applied to determination of the presence or absence of a target nucleic acid molecule, the signal-generating process may be performed in accordance with a multitude of methods known to one of skill in the art. The methods include TaqMan™ probe method (U.S. Pat. No. 5,210,015), Molecular Beacon method (Tyagi et al., Nature Biotechnology, 14 (3):303(1996)), Scorpion method (Whitcombe et al., Nature Biotechnology 17:804-807(1999)), Sunrise or Amplifluor method (Nazarenko et al., Nucleic Acids Research, 25(12):2516-2521(1997), and U.S. Pat. No. 6,117,635), Lux method (U.S. Pat. No. 7,537,886), CPT (Duck P, et al., Biotechniques, 9:142-148(1990)), LNA method (U.S. Pat. No. 6,977,295), Plexor method (Sherrill CB, et al., Journal of the American Chemical Society, 126:4550-4556(2004)), Hybeacons™ (D. J. French, et al., Molecular and Cellular Probes (2001) 13, 363-374 and U.S. Pat. No. 7,348,141), Dual-labeled, self-quenched probe (US 5,876,930), Hybridization probe (Bernard PS, et al., Clin Chem 2000, 46, 147-148), PTOCE (PTO cleavage and extension) method (WO 2012/096523), PCE-SH (PTO Cleavage and Extension-Dependent Signaling Oligonucleotide Hybridization) method (WO 2013/115442) and PCE-NH (PTO Cleavage and Extension-Dependent Non-Hybridization) method (PCT/KR2013/012312) and CER method (WO 2011/037306).

The term used herein "amplification" or "amplification reaction" refers to a reaction for increasing or decreasing signals. The increase or decrease of signals occurs from the signal-generating means.

According to an embodiment of this invention, signals from the signal-generating means are generated depending on the presence of the analyte (*e.g.,* target nucleic acid molecule) and their intensities are increased or decreased upon the course of the amplification reaction.

According to an embodiment, the amplification reaction means a reaction for amplifying signals from the signal-generating means depending on the presence of the analyte (e.g., target nucleic acid molecule).

According to an embodiment, an amplification curve is obtained by the amplification reaction.

The term used herein "cycle" refers to a unit of changes of conditions in a plurality of measurements accompanied with changes of conditions. For example, the changes of conditions include changes in temperature, reaction time, reaction number, concentration, pH and/or replication number of a measured subject (*e.g.*, target nucleic acid molecule). Therefore, the cycle may include time or process cycle, unit operation cycle and reproductive cycle.

For instance, when a substrate decomposition capacity by an enzyme is analyzed depending on concentrations of the substrate, a plurality of measurements for the decomposition capacity by the enzyme is carried out with varying substrate concentrations. The increases in the substrate concentration may correspond to the changes of conditions and a unit of the increases may correspond to a cycle.

As another example, an isothermal amplification allows for a plurality of measurements for a sample in the course of reaction time under isothermal conditions and the reaction time may correspond to the changes of conditions and a unit of the reaction time may correspond to a cycle.

Particularly, when repeating a series of reactions or repeating a reaction with a time interval, the term "cycle" refers to a unit of the repetition.

For example, in a polymerase chain reaction (PCR), a cycle refers to a reaction unit comprising denaturation of a target molecule, annealing (hybridization) between the target molecule and primers and primer extension. The increases in the repetition of reactions may correspond to the changes of conditions and a unit of the repetition may correspond to a cycle.

According to an embodiment, where the target nucleic acid molecule is present in a sample, values (e.g., intensities) of signals measured are increased or decreased upon increasing cycles of an amplification reaction.

According to an embodiment, the amplification reaction to amplify signals indicative of the presence of the target nucleic acid molecule is performed in such a manner that signals are amplified simultaneously with amplification of the target nucleic acid molecule (e.g., real-time PCR). Alternatively, the amplification reaction is performed in such a manner that signals are amplified with no amplification of the target nucleic acid molecule [e.g., CPT method (Duck P, et al., Biotechniques, 9:142-148 (1990)), Invader assay (U.S. Pat. Nos. 6,358,691 and 6,194,149)].

A multitude of methods have been known for amplification of a target nucleic acid molecule, including, but not limited to, PCR (polymerase chain reaction), LCR (ligase chain reaction, see Wiedmann M, et al., "Ligase chain reaction (LCR)- overview and applications." PCR Methods and Applications 1994 Feb;3(4):S51-64), GLCR (gap filling LCR, see WO 90/01069, EP 439182 and WO 93/00447), Q-beta (Q-beta replicase amplification, see Cahill P, et al., Clin Chem.,

37(9):1482-5(1991), U.S. Pat. No. 5556751), SDA (strand displacement amplification, see G T Walker et al., Nucleic Acids Res. 20(7):16911696(1992), EP 497272), NASBA (nucleic acid sequence-based amplification, see Compton, J. Nature 350(6313):912(1991)), TMA (Transcription-Mediated Amplification, see Hofmann WP et al., J Clin Virol. 32(4):289-93(2005); U.S. Pat. No. 5888779).) or RCA (Rolling Circle Amplification, see Hutchison C.A. et al., Proc. Natl Acad. Sci. USA. 102:1733217336(2005)).

According to an embodiment, the label used for the signal-generating means may be a fluorescent label, more particularly, a fluorescent single label or an interactive dual label containing a fluorescent reporter molecule and a quencher molecule. According to an embodiment, the amplification reaction used in the present invention amplifies signals simultaneously with amplification of the target nucleic acid molecule. According to an embodiment, the amplification reaction is performed in accordance with PCR.

The signal-generating process provides a data set (*e.g.,* values of signals and cycles) for analyzing the sample.

The term used herein "values of signals" means either values of signals actually measured at the cycles of the signal-generating process (*e.g.,* actual value of fluorescence intensity processed by amplification reaction) or their modifications. The modifications may include mathematically processed values of measured signal values (*e.g.,* intensities). Examples of mathematically processed values of measured signal values may include logarithmic values and derivatives of measured signal values. The derivatives of measured signal values may include multi-derivatives.

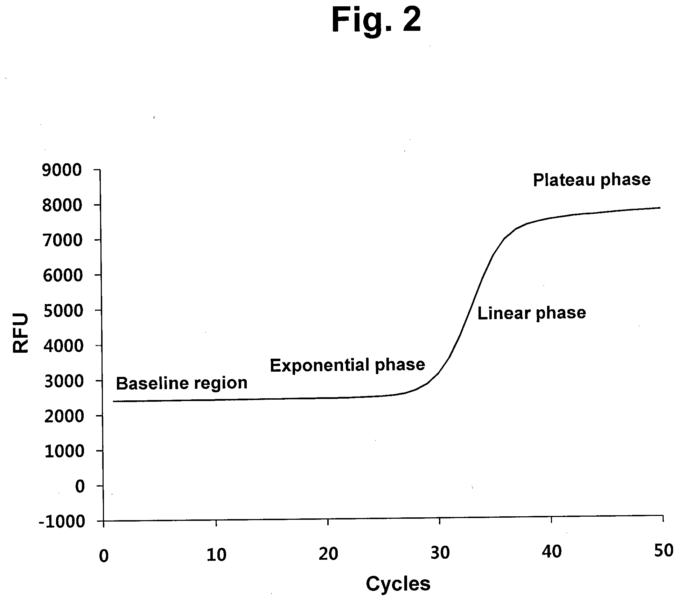

The term used herein "data point" means a coordinate value comprising a cycle and a value of signal at the cycle.Data points obtained by the amplification reaction using the signal-generating means may be plotted with coordinate values in a rectangular coordinate system. In the rectangular coordinate system, the X-axis represents cycles of the amplification reaction and the Y-axis represents values of signals from the signal-generating means at the cycles (*e.g.,* Fig. 2).

The term used herein "data set" refers to a set of data points. The data set comprises the raw data set and the modified data set.

Raw data set includes a preliminary data set for the analysis of the present application. The raw data set may include a set of data points obtained directly from the signal-generating process (*e.g.,* an amplification reaction) for the sample analysis.

For example, where the present invention is used for correcting a raw data set of an amplification reaction, the raw data set may include a set of data points obtained directly from the amplification reaction (*e.g.,* Fig. 2).

Modified data set includes a mathematically processed data set of the raw data set. The modified data set include a corrected data set and slope data set. The corrected data set is a set of data points obtained by correction of the raw data set.

In the Specification, the raw data set and the modified data set may have relative meanings. For instance, the raw data set may refer to a data set prior to any modification of data and the modified data set may refer to a data set obtained after modification(s) of data.

The data set used in the present invention may comprise a portion or all of the data points obtained from the signal-generating process or a portion or all of the corrected data points.

According to an embodiment of this invention, the signal-generating process is a process with amplification of the target nucleic acid molecule. More particularly, the process with amplification of the target nucleic acid molecule is real-time polymerase chain reaction (real-time PCR).

According to an embodiment of the invention, the values of signals are values of signals generated from the signal-generating process or mathematically modified values of the signals generated from the signal-generating process.

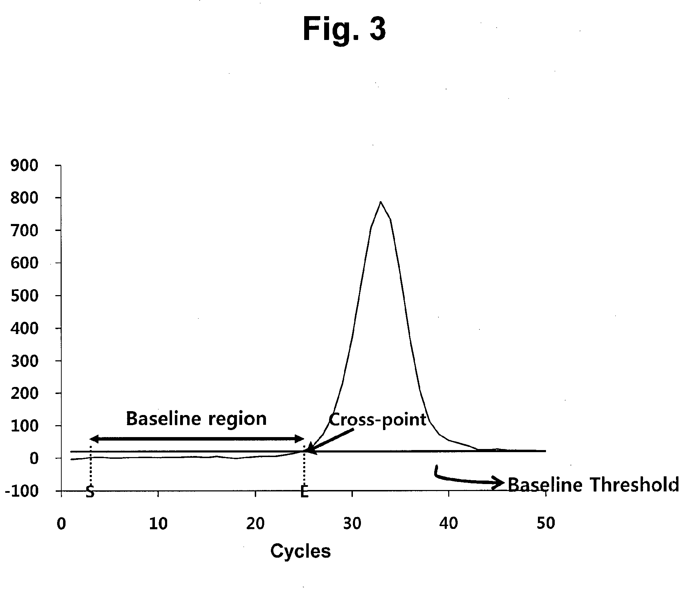

According to an embodiment of this invention, the signal-generating process is real-time PCR, the value of signals are mathematically modified values of signals generated from the real-time PCR, and the mathematically modified values are obtained by differentiating the values of signals with respect to the cycles (see Fig. 3). The differentiated values of signals with respect to the cycles include derivatives of the raw data as described above.

Fig. 2 represents a specific example of a data set obtained from real-time PCR as a signal-generating process and corresponds to an amplification curve of real-time PCR. The data set presented in Fig. 2 is a raw data set obtained directly from the signal-generating process (real-time PCR). The raw data set comprises amplification cycles of real-time PCR and signal intensities (e.g., RFU) measured at the amplification cycles.

Fig. 3 represents one of modifications of the raw data set, which contains data points of slopes calculated at the amplification cycles. The curve of Fig. 3 corresponds to a derivative of the raw data of Fig. 2.

An amplification curve representing the amplification reaction may be obtained by plotting values of signals against amplification cycles. The amplification curves herein refer to curves obtained by plotting the data set.

The pre-corrected amplification curve refers to an amplification curve before correction showing values of signals measured at each amplification cycle or their modifications, which is based on values of signals measured or their modifications. The pre-corrected amplification curve may be obtained by plotting signal intensities measured against amplification cycles. Particularly, the pre-corrected amplification curve may be obtained by plotting the raw data set.

The corrected amplification curve refers to an amplification curve corrected based on the pre-corrected amplification curve. The corrected amplification curve may be obtained by plotting a corrected data set.

The term used herein "target nucleic acid" or "target nucleic acid molecule" refers to a nucleic acid molecule of interest for detection or quantification. The target nucleic acid molecule comprises a sequence in a single strand as well as in a double strand. The target nucleic acid molecule comprises a sequence initially present in a nucleic acid sample as well as a sequence newly generated in reactions.

The target nucleic acid molecule may include any DNA (gDNA and cDNA), RNA molecules their hybrids (chimera nucleic acid). The molecule may be in either a double-stranded or single-stranded form. Where the nucleic acid as starting material is double-stranded, it is preferred to render the two strands into a single-stranded or partially single-stranded form. Methods known to separate strands includes, but not limited to, heating, alkali, formamide, urea and glycoxal treatment, enzymatic methods (e.g., helicase action), and binding proteins. For instance, strand separation can be achieved by heating at temperature ranging from 80°C to 105°C. General methods for accomplishing this treatment are provided by Joseph Sambrook, et al., Molecular Cloning, A Laboratory Manual, Cold Spring Harbor Laboratory Press, Cold Spring Harbor, N.Y.(2001).

The target nucleic acid molecule includes any naturally occurring prokaryotic, eukaryotic (for example, protozoans and parasites, fungi, yeast, higher plants, lower and higher animals, including mammals and humans), viral (for example, Herpes viruses, HIV, influenza virus, Epstein-Barr virus, hepatitis virus, polio virus, etc.), or viroid nucleic acid. The nucleic acid molecule can also be any nucleic acid molecule which has been or can be recombinantly produced or chemically synthesized. Thus, the nucleic acid sequence may or may not be found in nature. The target nucleic acid molecule may include known or unknown sequences.

The term used herein "sample" refers to any cell, tissue, or fluid from a biological source, or any other medium that can advantageously be evaluated according to this invention, including virus, bacteria, tissue, cell, blood, serum, plasma, lymph, sputum, swab, aspirate, bronchoalveolar lavage fluid, milk, urine, faeces, ocular fluid, saliva, semen, brain extracts, spinal cord fluid (SCF), appendix, spleen and tonsillar tissue extracts, amniotic fluid, ascitic fluid and non-biological samples (*e.g.,* food and water). The sample also includes solution or solid substance for chemical reaction. In addition, the sample includes natural-occurring nucleic acid molecules isolated from biological sources and synthetic nucleic acid molecules.

**Step (b): Applying Threshold Value to Each of Cycles (S20)**

A threshold value is applied to each of the cycles such that a plurality of threshold values are applied to the cycles. The threshold values of at least two cycles among the cycles are different from each other. In other words, the plurality of threshold values have wholly or partially different values from each other.

The most prominent feature of the present invention is to apply a plurality of threshold values to the cycles of the signal-generating process in which the threshold values of at least two cycles among the cycles are different from each other.

Each cycle is assigned with one individual threshold value. For example, when the number of cycles of a signal-generating process is thirty (30), the threshold values in the number of thirty (30) are assigned individually. The assigned threshold values may be the same or different from each other. The most striking feature of the present invention is that at least two among the assigned threshold values are different from each other.

The application of threshold values are conducted for selecting data points satisfying threshold criteria determined by the threshold values. Conventionally, a single threshold value has been adopted for evaluating values of signals from a signal-generating process. In other words, the conventional technologies suggested hitherto have employed fixed threshold methods using an identical threshold value over all cycles for evaluating values of signals from a signal-generating process.

Unlikely, the present invention utilizes a variable threshold of which the threshold values of at least two cycles among cycles are different from each other, thereby finally analyzing the sample.

The threshold values of at least two cycles among the cycles are different from each other. That is to say, the plurality of threshold values have wholly or partially different values from each other.

A graph obtained by plotting threshold values against cycles is named as TC graph (threshold cycle graph). The TC graph is a graph obtained by plotting a threshold set. The threshold set refers to a set of threshold points. The threshold point means a coordinate value comprising a cycle and a threshold value at the cycle.

A threshold value applied to a data set for obtaining a baseline is named as baseline threshold value and a graph obtained by plotting baseline threshold values against cycles is named as BT graph.

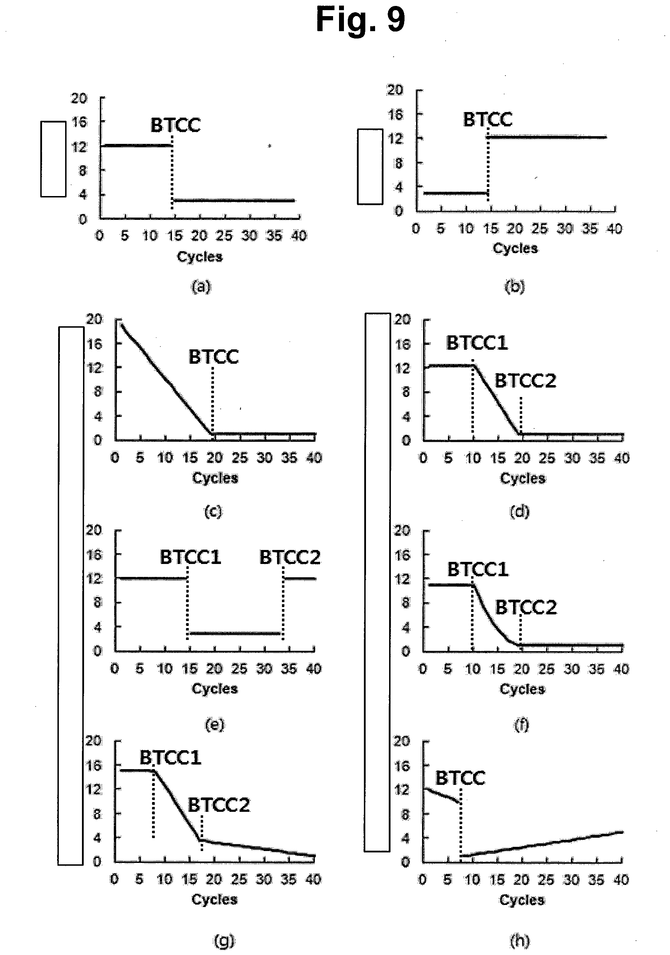

When the present method is used for correcting a raw data set of an amplification reaction (*e.g.*, baselining), the BT graph as one of the TC graphs is obtained by plotting baseline threshold values against cycles (see Fig. 9).

According to an embodiment, at least two cycles among the cycles have different threshold values from each other, thereby much more accurately obtaining information for analyzing a sample. This approach is named herein as "variable threshold (VT)" method. The VB method comprises variable baseline threshold and variable signal threshold method.

According to an embodiment, the threshold values are determined in such a manner that with respect to a threshold-changed cycle (TCC), a function formed by a set of pre-TCC cycles and threshold values to be applied to the pre-TCC cycles is different from a function formed by a set of post-TCC cycles and threshold values to be applied to the post-TCC cycles.

According to an embodiment, either the function for pre-TCC cycles or the function for post-TCC cycles may be applied to the TCC.

The term used herein "threshold-changed cycle (TCC)" refers to a benchmark cycle at which a pattern of change of threshold values is altered over cycles. In particular, the term "threshold-changed cycle (TCC)" refers to a benchmark cycle at which a threshold value is changed. The TCC may exist in a singular or plural number. The term "pre-TCC cycles" refers to cycles before the TCC and the term "post-TCC cycles" to cycles after the TCC.

The expression in which a function formed by a set of pre-TCC cycles and threshold values to be applied to the pre-TCC cycles is different from a function formed by a set of post-TCC cycles and threshold values to be applied to the post-TCC cycles, means that the TC graphs for pre-TCC cycles and the post-TCC cycles exhibit different patterns from each other. Examples of the embodiment are represented by Fig. 9 of which descriptions are found in Section II as below.

The TCC may be established in the number of one or more for a reaction.

According to an embodiment, the number of TCC may be 1, 2, 3, 4, 5, 6, 7, 8, 9, 10, 20, 30, 40 or 50. The number of TCC may be not more than 70, 60, 50, 40 or 30. Particularly, the number of TCC may be 1-2 or 1-3.

According to an embodiment, the cycles are classified into at least two different groups in terms of at least one threshold-changed cycle (TCC). Cycles classified into a group are continuous, and have the same threshold value. Cycles classified into immediately adjacent-different groups have different threshold values from each other. Therefore, cycles classified into distantly different groups have different or same threshold values. The TCC may have the same threshold value as that for cycles before or after the TCC. The TCC may be established in the number of one for a data set such that cycles of the data set may be classified into two groups. Alternatively, the TCC may be established in the number of not less than two for a data set such that cycles of the data set may be classified into not less than three groups.

Example of the expression "cycles classified into immediately adjacent-different groups" is as follows: The signal-generating process comprises a total of 40 cycles, Group 1 is in a range of cycles 1-10, Group 2 is in a range of cycles 11-20, Group 3 is in a range of cycles 21-30 and Group 4 is in a range of cycles 31-40. The immediately adjacent-different groups are Groups 1 and 2, Groups 2 and 3, or Groups 3 and 4. The distantly different groups are Groups 1 and 4, Groups 1 and 3, or Groups 2 and 4.

According to an embodiment, the functions of the immediately adjacent-different groups are different from each other, and the function of the distantly different groups are different or same each other.

According to an embodiment, the step (b) further comprises applying an additional threshold value to at least one cycle among the cycles.

According to an embodiment, an additional threshold set is applied to a data set.

According to an embodiment, a threshold set is a variable threshold set in which threshold values applied to at least two cycles among the cycles are different from each other.

According to an embodiment, a threshold set is a fixed- threshold set in which threshold values applied to the cycles have the same one.

According to an embodiment, multiple threshold sets comprise at least two threshold sets selected from the group consisting of variable-threshold sets and fixed-threshold sets.

According to an embodiment, multiple threshold sets comprise at least one fixed-threshold set.

According to an embodiment, multiple threshold sets comprise at least one variable-threshold set.

According to an embodiment, multiple threshold sets comprise at least one fixed- threshold set and at least one variable-threshold set.

According to an embodiment, each of multiple threshold sets has a corresponding threshold criterion.

According to an embodiment, multiple threshold sets are simultaneously applied for analyzing a data set. When a plurality of cycles satisfying a threshold criterion are observed, all or a portion of them may be used for analysis.

According to an embodiment, multiple threshold sets are sequentially applied for analyzing a data set. When cycles satisfying a threshold criterion of a firstly applied threshold set are not observed, another threshold set is then applied.

According to an embodiment, a threshold set is applied to a data set and an additional threshold value is applied to at least one cycle among the cycles.

The application of the additional threshold value can be described with reference to descriptions for (3) Approach to multiple baseline threshold value of Section II discussed below.

### Step (c): <u>Identifying Cycles Satisfying Threshold Criterion (S30)</u>

Following application of the threshold value to each cycle, one or more cycles satisfying a threshold criterion determined by each of the threshold values are identified.

The term used herein "threshold criterion" refers to a criterion for identification of cycles having a certain characteristic, which is determined by each of the threshold values.

According to an embodiment, the threshold criterion may be any reference or benchmark comprising a value of signal at the cycle of interest and a threshold value.

According to an embodiment, the threshold criterion may be magnitude relation between a value of signal at a cycle and a threshold value applied to the cycle.

According to an embodiment, the threshold criterion is to compare value of a signal for each of the cycles with a threshold value to each of the amplification cycles.

Particularly, the threshold criterion is defined by that a value of signal is not less than or not more than the threshold value.

According to an embodiment, the threshold criterion is to have a value of signal the same as or more than the threshold value.

For example, where the threshold values are established as 5 in a range of cycles 1-10 and 2 in a range of cycles 11-20, the threshold criterion may be defined by values of signals of not less than 5 in a range of cycles 1-10 and not less than 2 in a range of cycles 11-20.

### Step (d): <u>Analyzing Sample Using the Identified Cycle or Cycles(S40)</u>

The sample is analyzed by using the identified cycle or cycles in the step (c).

According to an embodiment, the analyzing the sample is to determine the presence of a target nucleic acid molecule in the sample and the identifying one or more cycles satisfying the threshold criterion is to determine $C_t$ value. In this case, the threshold criterion may be to have a value of signal the same as a threshold value. The number of cycles to be identified may be one.

Where the present method is applied to determining $C_t$ value in real-time PCR for determination of the presence of a target nucleic acid molecule, the present method comprises the steps of:

(a) obtaining a value of signal at each of cycles of real-time PCR using the sample to provide values of signals at the cycles;
(b) applying a signal threshold value to each of the cycles such that a plurality of signal threshold values are applied to the cycles; wherein the signal threshold values of at least two cycles among the cycles are different from each other;
(c) identifying a cycle satisfying a threshold criterion determined by each of the signal threshold values; and
(d) determining $C_t$ value in real-time PCR by using the identified cycle in the step (c).

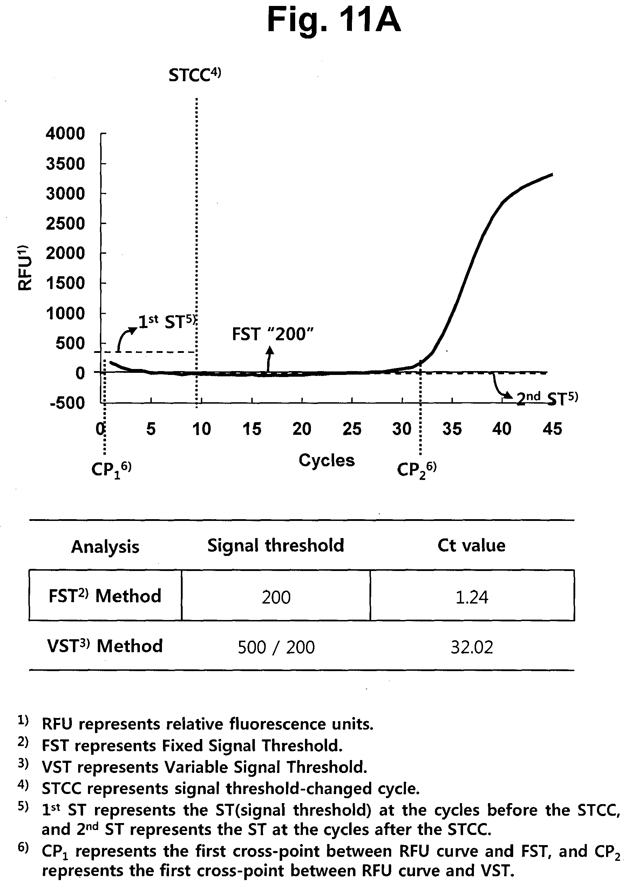

The application to determination of $C_t$ value in real-time PCR is exemplified in Example 3 and Figs. 11a-11c. The threshold value is re-named as a signal threshold value in determination of $C_t$ value. The present method for determination of $C_t$ value is called herein as VST (variable signal threshold) method.

As addressed in Example 3 and Figs. 11a-11c, the present method using variable signal threshold values can eliminate errors in which data points generating initial noise signals in early amplification cycles are determined as the presence of a target nucleic acid molecule. Furthermore, the present method is capable of determining more accurately a start-point of signal increase in later amplification cycles, thereby eliminating errors in determination of $C_t$ value.

According to an embodiment, the analyzing the sample is to determine the presence of a target nucleic acid molecule in the sample and the identifying one or more cycles satisfying the threshold criterion may be to determine an end-point cycle of a baseline region of an amplification curve of real-time PCR. In this case, the threshold criterion is to have a value of signal the same as a baseline threshold value. The number of cycles to be identified may be one.

Where the present method is applied to determining an end-point cycle of a baseline region of an amplification curve for determination of the presence of a target nucleic acid molecule, the present method comprises the steps of:

(a) obtaining a value of signal at each of cycles of real-time PCR using the sample to provide values of signals at the cycles such that a raw data set containing (i) amplification cycles of the real-time PCR and (ii) the values of signals at the amplification cycles is obtained;
(b) determining a baseline region by determining both a start-point cycle and an end-point cycle of the baseline region using the raw data set; wherein the end-point cycle is determined by the steps:

(b1) applying a baseline threshold value to each of the amplification cycles; such that a plurality of baseline threshold values are applied to the amplification cycles; wherein the baseline threshold values of at least two cycles among the cycles are different from each other; and

(b2) identifying one or more cycles satisfying a baseline threshold criterion determined by each of the baseline threshold values;

(b3) determining the end-point cycle of the baseline region by using the identified cycle or cycles in the step (b2);

(c) establishing a function for a best-fit line of the baseline region using at least two data points of the raw data set within the baseline region; and

(d) obtaining a corrected data set by subtracting values of the function for the best-fit line from the values of the signals of the raw data set; wherein the corrected data set contains (i) the amplification cycles of the real-time PCR and (ii) the resultants of the subtraction.

More particularly, the identification in the step (b2) is performed by comparing a slope calculated for each of the amplification cycles using the raw data set with a baseline threshold value for each of the amplification cycles.

Since the method described in Section II is a representative example of this application in Section I, the common descriptions between them are omitted in order to avoid undue redundancy leading to the complexity of this specification.

The present method may be applied to signal changes with any pattern including signal change with an increased pattern (e.g., signal change by amplification reactions) and signal change with a decreased pattern.

## II. Correction of Raw Data Set of Amplification Reaction

In another aspect of this invention, there is provided a method for correcting a raw data set of an amplification reaction using a signal-generating means, comprising:

(a) obtaining the raw data set containing (i) amplification cycles of the amplification reaction and (ii) values of signals obtained from the signal-generating means at the amplification cycles;

(b) determining a baseline region by determining both a start-point cycle and an end-point cycle of the baseline region using the raw data set;

(c) establishing a function for a best-fit line of the baseline region using at least two data points of the raw data set within the baseline region; and

(d) obtaining a corrected data set by subtracting values of the function for the best-fit line from the values of the signals of the raw data set; wherein the corrected data set contains (i) the amplification cycles of the amplification reaction and (ii) the resultants of the subtraction.

Since the present method for method for correcting a raw data set of an amplification reaction is a particular embodiment of the present method for analyzing a sample, the common descriptions between them are omitted in order to avoid undue redundancy leading to the complexity of this specification.

The present inventors have made intensive researches to develop novel approaches for correcting a raw data set of an amplification reaction, thereby qualitatively or quantitatively providing analysis results of an amplification reaction in a more accurate and reliable manner. As results, we have found novel approaches for correcting a raw data set of an amplification reaction. In particular, we have found that an end-point cycle of a baseline region can be determined by our novel methods, contributing to obtaining analysis results of an amplification reaction in a more accurate and reliable manner.

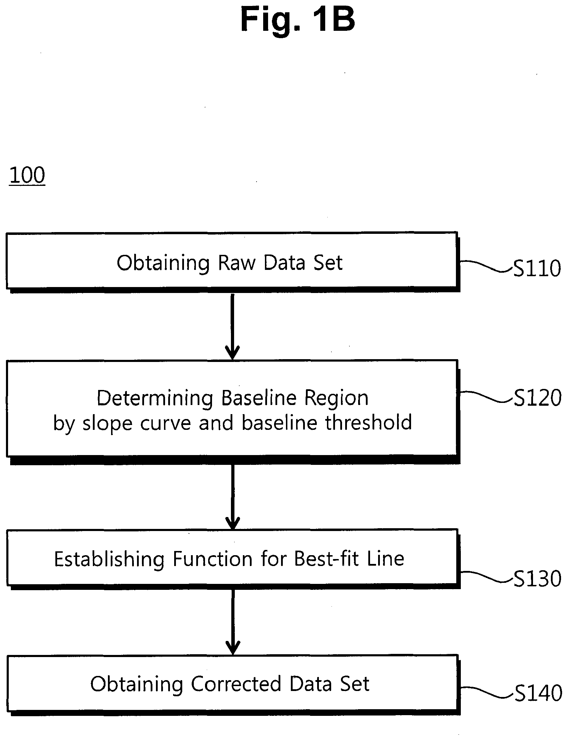

Fig. 1b represents a flow diagram illustrating an embodiment of the present method for correcting a raw data set of an amplification reaction. The present invention will be described in more detail as follows:

### Step (a): Obtaining a Raw Data Set(S110)

First, a raw data set is obtained. The raw data set contains (i) amplification cycles of the amplification reaction and (ii) values of signals obtained from the signal-generating means at the amplification cycles.

The raw data is obtained by performing the amplification reaction using the signal-generating means. The step (a) may be alternatively expressed as obtaining a raw data set by performing an amplification reaction for a sample using the signal-generating means.

According to an embodiment of this invention, signals from the signal-generating means are generated depending on the presence of the target nucleic acid molecule and their intensities are increased or decreased upon the course of the amplification reaction.

According to an embodiment, the amplification reaction means a reaction for amplifying signals from the signal-generating means depending on the presence of the target nucleic acid molecule.

Particularly, when repeating a series of reactions or repeating a reaction with a time interval, the term "cycle" refers to a unit of the repetition.

For example, in a polymerase chain reaction (PCR), a cycle refers to a reaction unit comprising denaturation of a target molecule, annealing (hybridization) between the target molecule and primers and primer extension. The increases in the repetition of reactions may correspond to the changes of conditions and a unit of the repetition may correspond to a cycle. As another example, for isothermal nucleic acid amplification as LAMP (Loop-mediated isothermal amplification) and NASBA (Nucleic acid sequence-based amplification), a cycle refers to a time interval.

According to an embodiment, where the target nucleic acid molecule is present in a sample, values (*e.g.*, intensities) of signals measured are increased or decreased upon increasing an amplification cycle number.

The raw data set comprises (i) amplification cycles and (ii) values of signals obtained from the signal-generating means at the amplification cycles.

The term used herein "values of signals" means either values of signals actually measured at the amplification cycles or their modifications. The modifications may include mathematically processed values of measured signal values (*e.g.,* intensities). Examples of mathematically processed values of measured signal values may include logarithmic values and derivatives of measured signal values. The derivatives of measured signal values may include multi-derivatives.

As described above, in the step for obtaining the raw data set (S110), a data set containing amplification cycles and values of signals from the signal-generating means at the amplification cycles is obtained by performing the amplification reaction, and plotted to provide the pre-corrected amplification curve (a first amplification curve).

The raw data set containing (i) amplification cycles and (ii) values of signals at the amplification cycles is obtained by the amplification reaction, and plotted to provide a pre-corrected amplification curve as illustrated in Fig. 2. In Fig. 2, RFU represents a relative fluorescence unit.

As described above, the amplification curve may be classified into a baseline region, an exponential phase, linear phase and a plateau phase. In the baseline region, there is little change in fluorescent signals during initial cycles of amplification. The exponential phase shows increase in fluorescent signals in proportional to increase of amplification products. In the linear phase, the increase in fluorescent signals is substantially reduced and behaves in a substantially linear manner. In the plateau phase, there is little increase in fluorescent signals due to saturation of both amplification products and fluorescent signals.

Because a background signal mostly occupying fluorescent signals in a baseline region results to baseline drift regardless of the amount of nucleic acid molecules in a sample, determining a baseline region and correcting an amplification curve have to be made.


**Step (b): Determining Baseline Region(S120)**

Afterwards, the baseline region is determined by determining both a start-point cycle and an end-point cycle of the baseline region using the raw data set.

The phrase "using the raw data set" with reference to determination of the baseline region is used to intend to encompass direct and indirect use of the raw data set. The indirect use of the raw data set includes use of the modified data set of the raw data.

According to an embodiment, both the start-point cycle and the end-point cycle may be determined directly from the raw data set or from mathematically processed data set of the raw data set.

For instance, the start-point cycle may be determined directly from the raw data set by determining a first cycle having a value of signal not less than a certain value. Alternatively, the end-point cycle may be determined from mathematically processed data set by determining a first cycle having a slope value not less than a certain value in which the slope value is obtained by mathematical processing of the raw data set.

The term "start-point cycle" means a cycle corresponding to the start of the baseline region.

The start-point cycle (S) of the baseline region may be arbitrarily determined by users. In general, the start-point cycle may be determined with a cycle after cycles showing a typical variation behavior during early amplification reactions. For example, the start-point cycle may be determined within cycles 1-10, *e.g.,* 2-10, 2-8, 2-6 or 2-4 cycles.

Alternatively, the start-point cycle (S) of the baseline region may be determined in considering cycles satisfying certain criteria.

For example, the start-point cycle may be determined with a first cycle showing a slope trend different from prior cycles. The cycle showing a slope trend different from prior cycles includes, for example, a cycle having a slope larger than prior cycles and less than 10% than a slope of an initial cycle, a cycle having a positive numbered slope when prior cycles have negative numbered slope, or a cycle having a negative numbered slope when prior cycles have positive numbered slope.

According to an embodiment, a ratio of change in signal value is calculated at each cycle and used for determining either the start-point cycle or an end-point cycle of the baseline region. Unless otherwise indicated, the term "slope" refers to a ratio of change in signal value at a selected cycle. According to an embodiment, slopes are plotted against cycles to provide a slope curve.

The term "end-point cycle" means a cycle corresponding to the termination of the baseline region. Since the end-point cycle (E) of the baseline region determines the end of the baseline region, it may be determined with a cycle prior to occurrence of signal amplification.

The end-point cycle of the baseline region may be determined from the raw data set or its modified data set.

The end-point cycle of the baseline region may be determined by various approaches.

For instance, the end-point cycle may be determined with a cycle exhibiting maximum second derivative of a data set. Alternatively, characteristics (e.g., location and size) of a slope curve of a data set may be analyzed to determine the end-point cycle. For example, as a peak of an exponential region has the highest, the end-point cycle of the baseline region may be determined with a start cycle of the peak of an exponential region.

Furthermore, the end-point cycle may be determined with a first cycle exhibiting a sharp increase in coefficient of variation compared with prior cycles. Alternatively, the end-point cycle may be determined with a cycle having coefficient of variation more than a predetermined value. The coefficient of variation may be defined as the ratio of the standard deviation to the mean. The coefficient of variation may be calculated in such a manner that a cycle whose coefficient of variation is calculated and cycles in a certain number before and after the cycle are selected and the standard deviation to the mean for signals at then the selected cycles are calculated for obtaining the coefficient of variation. The certain number of the selected cycles may be one, two, three, four or five, particularly one or two.

According to an embodiment, the end-point cycle is determined from the start-point cycle to a cycle of a data point having the highest slope among the data set.

According to an embodiment, the end-point cycle is determined with a cycle selected among not-less-than cycles. Alternatively, the end-point cycle is determined in such a manner that a cycle selected among not-less-than cycles is used as a reference cycle for determining the end-point cycle.

The term used herein "not-less-than cycle(s)" means a cycle or cycles of data point(s) having a value equal to or more than a baseline threshold value. In other words, the not-less-than cycles include cycles having slopes not less than a baseline threshold value.

When the amplification curve shows a decrease pattern, the end-point cycle may be determined with a cycle selected among not-more-than cycles.

According to an embodiment, the end-point cycle is determined with a cycle selected among cross-point cycles. Alternatively, the end-point cycle is determined in such a manner that a cycle selected among cross-point cycles is used as a reference cycle for determining the end-point cycle.

The term used herein "cross-point cycle(s)" means a cycle or cycles of data point(s) having a value equal to a baseline threshold value.

The cross-point cycle may be determined with one among cycles of data points.

The cross-point cycle may be determined with a cycle that is mathematically calculated using data points and a baseline threshold value.

According to an embodiment, the end-point cycle is determined with a cycle of data point(s) having a slope equal to a baseline threshold value, with a cycle of a first data point having a slope more than a baseline threshold value or with a cycle of data point(s) having the first slope value among slopes less than a baseline threshold value. Alternatively, the end-point cycle is determined in such a manner that a cycle of a data point having a slope with a certain value or a cycle of a first data point exceeding a slope with a certain value is used as a reference cycle and then applied to a mathematical equation for determining the end-point cycle. Examples of the mathematical equation include "the end-point cycle = the reference cycle - (1, 2, 3 or 4 cycles)"; "the end-point cycle = the reference cycle + (1, 2, 3 or 4 cycles)"; "the end-point cycle = [the reference cycle x 0.9]"; and "the end-point cycle = [the reference cycle - (baseline threshold value x 0.1)]". [X] denotes the greatest integer that is less than or equal to X.

The certain value described with conjunction with the slope is a baseline threshold value (BT). The baseline threshold value is generally used to establish a baseline and for this invention to determine the end-point cycle of a baseline region. The baseline threshold value may be predetermined (or input) depending on subjects of measurements and/or measurement devices, or arbitrarily determined by users.

According to an embodiment, the end-point cycle is determined with a cycle of a first cross-point between a baseline threshold and a slope curve or a cycle most adjacently to a cycle of a data point of a first cross-point.

The cycle of the cross-point or the cycle of the data point of the cross-point is described herein as a cross-point cycle. The cross-point cycle may be described as a cycle of a cross-point between a slope curve and a graph (named as baseline threshold graph or BT graph) obtained by plotting baseline threshold value(s) against each cycle.

The number of the cross-point cycle may be one or more than one depending on shapes of the slope curve and/or the BT graph. The cross-point cycle for determining the end-point cycle may be determined with a predetermined certain cross-point cycle such as a first cross-point cycle or a last cross-point cycle. Alternatively, when the number of the cross-point cycle may be not less than two, a cross-point cycle having the lowest cycle number may be determined as the end-point cycle.

The numerical value of the cycle of the cross-point may not be integer. It is advantageous that the end-point cycle has an integer value, because cycles are expressed as integer values in practical experiments. Therefore, a first integral cycle exceeding the cross-point cycle or a cycle at 1, 2, 3 or 4 cycles before or after the first integral cycle may be determined as the end-point cycle. Alternatively, the end-point cycle may be determined with a maximum integral cycle less than the cross-point cycle or a cycle at 1, 2, 3 or 4 cycles before or after the maximum integral cycle.

According to an embodiment, the end-point cycle of a baseline region is determined with a cycle of a first cross-point between a baseline threshold value and a slope curve or a cycle at 1, 2, 3 or 4 cycles before or after a cycle of a data point of the first cross-point.

According to an embodiment, the baseline threshold value may be established such that the value is not interfered in a slope curve with a background signal during initial cycles before observing an exponential region.

According to an embodiment, the baseline threshold value may be established with a suitable value selected by analysis results for various samples.

Fig. 3 represents a baseline region in which a cycle among 2-4 cycles is determined as a start-point cycle and the first cross-point cycle between a baseline threshold and a slope curve is determined as an end-point cycle.

In the present invention, when a relative distance between the start-point cycle and the end-point cycle determined above is less than a certain value, the relative distance may be additionally adjusted to have a suitable baseline region.

The relative distance may be calculated by subtracting the start-point cycle from the end-point cycle. The certain value of the relative distance required to be adjusted may be 0, 1, 2, 3, 4, 5, 6, 7, 8, 9 or 10 cycles, particularly, 0, 1, 2, 3 or 4 cycles.

The additional adjustment may be performed in such a manner that either the start-point cycle or the end-point cycle, or both of them are arbitrarily adjusted to permit the relative distance to have more than the certain value. Alternatively, the additional adjustment may be performed in such a manner that either the start-point cycle or the end-point cycle is determined by the above-described method and then the other is determined to permit the relative distance to have more than the certain value.

According to an embodiment, a slope of a data point represents change in a value of signal at the cycle of the data point.

As described above, the amplification reaction in the present invention includes reactions exhibiting signal decrease over cycles.

The slope may be calculated by various approaches such as differentiation.

The slope may be calculated by a least square method or LMS (least mean square) algorithm using a data point of a certain cycle and at least one data point of a cycle or cycles before and/or after the certain cycle.

The below descriptions illustrate a least square method as a representative of a linear regression analysis but the scope of the present invention as set forth in the appended claims is not limited to the least square method.

The number of the data points used for slope calculation by the least square method may be not more than two. For example, the number of the data points may be not more than 2, 3, 4, 5, 6, 7, 8, 9, 10, 11, 12, 13, 14 or 15. Particularly, the number of the data points may be 2-3, 2-15, 3-11, 3-9, 3-7, 3-5 or 5-7.

The data points used for slope calculation by the least square method may be data points of adjacent cycles or data points of distal cycles.

For instance, the slope is calculated by a least square method using a data point of a certain cycle and at least one data point of a cycle or cycles before and/or after the certain cycle.

As another example, the slope is calculated by the least square method using the data point of the certain cycle, and a data point of a cycle before the certain cycle and a data point of a cycle after the certain cycle.

The number of the data points used for slope calculation by the least square method may be varied depending on cycles. For example, the slope of a data point may be calculated by the least square method using two or three data points of adjacent cycles. For example, because there are no cycles before the first cycle, the slope at the first cycle may be calculated by the least square method using two data points of the first and next cycles. The slope at the last cycle may be calculated by the least square method using two data points of the last cycle and an immediately preceding cycle because there are no cycles after the last cycle. For the other cycles, the slopes may be calculated by the least square method using the data point of a certain cycle, and a data point of a cycle just before the certain cycle and a data point of a cycle just after the certain cycle.

According to an embodiment, the least square method is expressed as the following mathematical equation 1:

## Equation 1

$$m = \frac{\sum_{i=I-a}^{I+b} (x_i - \bar{x})(y_i - \bar{y})}{\sum_{i=I-a}^{I+b} (x_i - \bar{x})^2}$$

wherein

$$\bar{x} = \frac{\sum_{i=I-a}^{I+b} x_i}{n} \qquad \bar{y} = \frac{\sum_{i=I-a}^{I+b} y_i}{n}$$

I is a cycle of a data point whose slope is to be calculated,
m is a slope of a data point at $I^{th}$ cycle,
$x_i$ is a cycle of $i^{th}$ cycle,
$y_i$ is a signal value measured at $i^{th}$ cycle,
n is a+b+1,
a and b independently represent an integer of 0-10 with a proviso that a is less than I, a+b+1 ranges from 2 to the number of data points of the raw data set and I+b is less than the number of data points of the raw data set.

The "a+b+1" is the number of data points used for calculating a slope at $I^{th}$ cycle, called as LSMR (Linear Squares Method Range). The "a" is a value for calculating a minimum cycle among a set of data points used for calculating a slope at $I^{th}$ cycle. The "b" is a value for calculating a maximum cycle. The number of data points refers to the data points obtained from the overall reaction, corresponding to the maximum cycle value of an amplification curve.

The "a" and "b" independently represent an integer of 0-10, particularly 1-5, more particularly 1-3.

Although it is advantageous that the values of "a" and "b" are the same, they may be different from each other depending on subjects of measurement, measurement environments and cycles.

It is advantageous that the "a" and "b" are applied to all data points of a reaction in non-varying manner, except for data points at which I-a is less than 1 or I+b is more than the number of all data points. Alternatively, slopes of a certain data point or a range of data points showing particular characteristics in considering variations of signal values and range characteristics may be calculated by applying different "a" and "b".

Even when the "a" and "b" are applied to all data points of a reaction in non-varying manner, the values of "a" and "b" different from those for the other data points may be applied for calculating slopes of data points at which I-a is less than 1 or I+b is more than the number of all data points. For instance, for data points at which I-a is less than 1, the "a" may be altered to permit "I-a" to become 1. At this time, the value of the "b" remains constant or changed upon altering the "a".

For data points at which I+b is more than the number of all data points, the "b" may be altered to permit "I+b " to be equal to the number of all data points. At this time, the value of the "a" remains constant or changed upon altering the "b".

The values of LSMR, "a" and "b" may be predetermined (or input) depending on subjects of measurements and/or measurement devices, or arbitrarily determined by users.

Fig. 3 represents a curve of slopes calculated by the least square method expressed by mathematical equation 1. Y-axis represents a slope of fluorescent signal intensities (or relative fluorescence unit) calculated for each of the amplification cycles by the least square method.

*End-point cycle in establishment of baseline region*

As described above, with avoiding interference by a background signal during initial cycles, the end-point cycle of the baseline region may be determined by comparing a slope calculated for each of the amplification cycles using the raw data set with a baseline threshold value to each of the amplification cycles.

The baseline threshold value is established for determining the end-point cycle of the baseline region. The baseline threshold value may be predetermined (or input) depending on subjects of measurements and/or measurement devices, or arbitrarily determined by users.

The amplification analysis faults by a background signal or noise are likely to due to erroneous determination of an end-point cycle. In amplification reactions such as nucleic acid amplification reactions, abnormal fluorescence signals during initial cycles are often detected and recorded. Detecting the abnormal fluorescence signals refers to detection of fluorescence signals not reflecting the amount of a target nucleic acid molecule.

When the baseline threshold value is established to be excessively low, slope values of the abnormal fluorescence signals may involve in determination of the end-point cycle. When a baseline region is established using such determined end-point cycle and then an amplification curve is corrected, the corrected amplification curve is very likely to be false positive and not to reflect the amount of amplicons. When the baseline threshold value is established to be excessively high for avoiding involvement of slope values of the abnormal fluorescence signals in determination of the end-point cycle, a cross between a baseline threshold and a slope curve may occur at later cycles rather than earlier cycles, or a cross between a baseline threshold and a slope curve may not occur when a peak of the slope curve is low.

According to an embodiment, cycles before a certain cycle are eliminated for solving the problem described above. Alternatively, a baseline threshold value is adjusted such that an initial background signal is not involved in determination of the end-point cycle.

According to an embodiment, the baseline threshold value may be established such that the value is not interfered with a background signal during initial cycles before observing an exponential region.

*(1) Approach to eliminate cycles before a certain cycle*

According to an embodiment, in a method to eliminate cycles before a certain cycle for determining the end-point cycle, the certain cycle is a minimum baseline end-point cycle (MBEC). In this method, the end-point cycle is determined with a cycle among cycles not less than the MBEC. By using the MBEC, it can be prevented that the end-point cycle is determined by cycles much earlier than an exponential region owing to a background or noise signal during initial cycles of amplification reactions. This approach in which the MBEC is established for determining the end-point cycle is named herein as "MBEC method".

The MBEC may be varied depending on patterns of a background or noise signal which are influenced by measurement apparatus, individual device characteristics of the apparatus, samples to be analyzed and reagents. The MBEC is not limited to a certain cycle range so long as faults due to a background or noise signal can be prevented. For example, the MBEC may be determined from cycles 1 to 50, particularly cycles 1-10, 1-15, 1-20, 1-25, 1-30, 1-35, 1-40, 5-10, 5-15, 5-20, 5-25, 5-30 more particularly cycles 5-15.

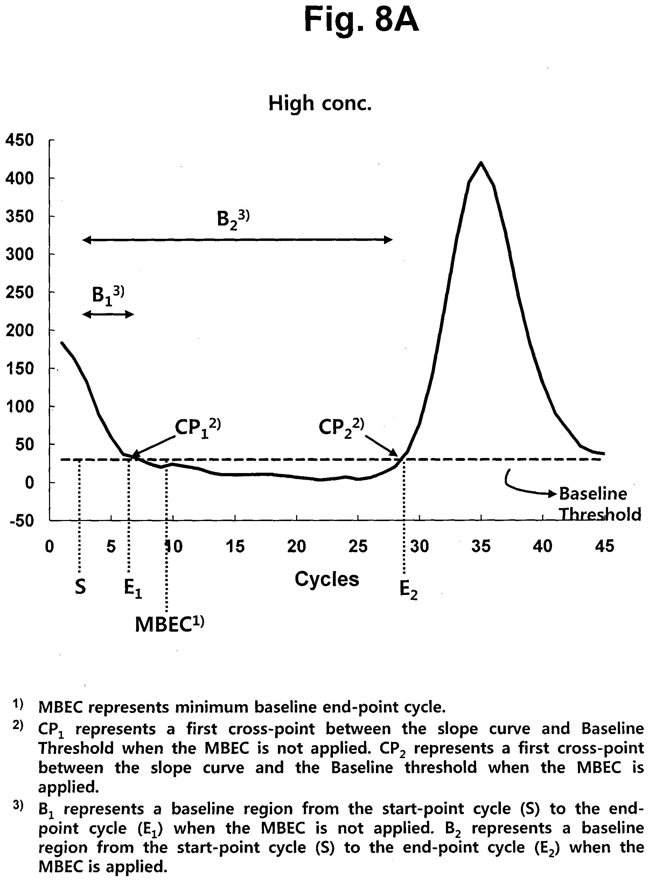

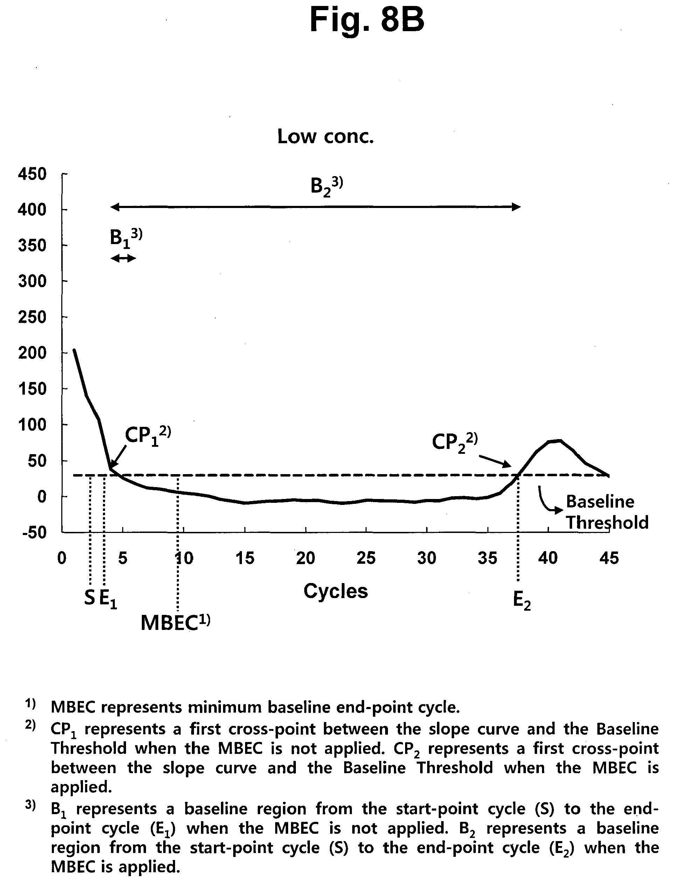

As illustrated in Figs. 8a and 8b, among the two cross-point cycles ($E_1$, $E_2$) of the slope curve, when the MBEC is applied, $E_2$ which is not less than the MBEC is determined as the end-point cycle.

Furthermore, when there are two or more cross-point cycles of the slope curve not less than the MBEC, lower cross-point cycle may be determined as the end-point cycle. As described above, when the numerical value of the least cross-point cycle not less than the MBEC is not integer, a first integral cycle exceeding the least cross-point cycle or a cycle at 1, 2, 3 or 4 cycles before or after the first integral cycle may be determined as the end-point cycle. Alternatively, the end-point cycle may be determined with a maximum integral cycle less than the least cross-point cycle or a cycle at 1, 2, 3 or 4 cycles before or after the maximum integral cycle.

According to an embodiment, the end-point cycle of the baseline region is determined with a cycle not less than a minimum baseline end-point cycle (MBEC) which may be determined before or after the amplification reaction.

After determining the MBEC, the end-point cycle of the baseline region may be easily and variously determined. For example, cross-point cycle(s) or not-less-than cycle(s) is first identified and then compared with the MBEC to evaluate whether the cycle is determined as the end-point cycle. Alternatively, the end-point cycle may be selected only from cycles after the MBEC.

When there are not cross-point cycles not less than the MBEC, the last cycle of the slope curve may be determined as the end-point cycle.

According to an embodiment, the end-point cycle of the baseline region is determined by a process comprising:

(i) obtaining a slope calculated for each of the amplification cycles;
(ii) comparing the slope with the baseline threshold value for each amplification cycle to obtain a candidate of the end-point cycle of the baseline region; and
(ii) comparing the candidate of the end-point cycle with the MBEC, wherein when the candidate of the end-point cycle is more than the MBEC, the candidate is determined as the end-point cycle.

When the candidate of the end-point cycle is less than the MBEC, the candidate is eliminated and then the steps (i) and (ii) are repeated to find a new candidate of the end-point cycle. When there is no candidate of the end-point cycle not less than the MBEC, the last cycle is determined as the end-point cycle.

The MBEC method of this invention can prevent to determine the end-point cycle with undesirable initial cycles of amplification reactions, resulting in obtaining more accurate correction of amplification curves.

*(2) Approach to variable baseline threshold value*

According to an embodiment, a baseline threshold (BT) value is adjusted such that an initial background signal is not involved in determination of the end-point cycle.

As described above, it is general to apply a fixed baseline threshold(FBT) value to all cycles of amplification reactions for determining the end-point cycle by comparing a slope calculated for each of the amplification cycles using the raw data set with a baseline threshold value to each of the amplification cycles.

According to the present invention, the baseline threshold value may be the same for each cycle or may be differently applied (or allocated) to different cycle groups. Alternatively, the baseline threshold value may be different for all cycles.

According to an embodiment, at least two cycles among the cycles have different baseline threshold values from each other. Therefore, a plurality of baseline threshold values have wholly or partially different values from each other. This approach in which the baseline threshold value applied to cycles are adjusted for allowing a plurality of baseline threshold values to have wholly or partially different values is named herein as "variable baseline threshold (VBT)" method.

According to an embodiment, the end-point cycle of the baseline region is determined by the steps:

(b1) applying a baseline threshold value to each of the amplification cycles such that a plurality of baseline threshold values are applied to the cycles; wherein the baseline threshold values of at least two cycles among the cycles are different from each other;
(b2) identifying one or more cycles satisfying a baseline threshold criterion determined by each of the baseline threshold values; and
(b3) determining the end-point cycle of the baseline region by using the identified cycle or cycles in the step (b2).

A graph obtained by plotting baseline threshold values against cycles is named as BT graph.

When a single baseline threshold value is allocated to all cycles, the BT graph has a straight line parallel to the x-axis.

According to the VBT method, various BT graphs with different baseline threshold values are obtained as represented in Fig. 9.

According to an embodiment, the cycles of the amplification reaction are classified into at least two different groups; wherein cycles classified into a group have the same threshold value, and cycles classified into different groups have different threshold values from each other. In such case, the BT graph has straight lines parallel to the x-axis (see Fig. 9, panels (a) and (b)).

According to an embodiment, the baseline threshold values for all or a portion of cycles may be increased or decreased at a certain ratio upon increasing cycles. In such case, the BT graph may be represented by a first order function (see Fig. 9, panels (c), (d), (g) and (h)).

According to an embodiment, the baseline threshold values for all or a portion of cycles may be increased or decreased at variable ratios upon increasing cycles. In such case, the BT graph may be represented by a curve function (e.g., second order function) (see Fig. 9, panel (f)).

According to an embodiment, the amplification cycles are classified into at least two different groups, cycles classified into the same group have the same baseline threshold value and cycles classified into different groups have different baseline threshold values. In such case, the BT graph may be represented by at least two functions. The BT graph may be plotted in a connected or disconnected manner.

According to an embodiment, a baseline threshold-changed cycle (BTCC) is established and different baseline threshold values are applied to cycles before and after the BTCC, respectively.

The term used herein "baseline threshold-changed cycle (BTCC)" means a benchmark cycle at which a pattern of change of baseline threshold values is altered over cycles. In particular, the term "baseline threshold-changed cycle (BTCC)" means a benchmark cycle at which a baseline threshold value is changed. The BTCC may be established in the number of one or more for an amplification reaction. The BTCC may be established before, during or after an amplification reaction.

According to an embodiment, the baseline threshold values for the amplification cycles are determined in such a manner that with respect to a baseline threshold-changed cycle (BTCC), a first function formed by a set of pre-BTCC cycles and baseline threshold values to be applied to the pre-BTCC cycles is different from a second function formed by a set of post-BTCC cycles and baseline threshold values to be applied to the post-BTCC cycles.

According to an embodiment, either the function for pre-BTCC cycles or the function for post-BTCC cycles may be applied to the BTCC.

As a baseline threshold value is applied to a cycle, a function of baseline threshold values and cycles may be formed. The function may be obtained using a set of baseline threshold values for all cycles or a portion of all cycles.

The expression in which a first function formed by a set of pre-BTCC cycles and baseline threshold values to be applied to the pre-BTCC cycles is different from a second function formed by a set of post-BTCC cycles and baseline threshold values to be applied to the post-BTCC cycles, means that the BT graphs for the first function of pre-BTCC cycles and the second function of post-BTCC cycles exhibit different patterns from each other.

For example, baseline threshold values applied to the pre-BTCC cycles may be represented by a first order function and baseline threshold values applied to the post-BTCC cycles may be represented by a constant function (see Fig. 9, panel (c)). In Fig. 9(c), the baseline threshold values for the pre-BTCC cycles are constantly decreased and those for the post-BTCC cycles are in a fixed value.

When there are two BTCCs, baseline threshold values for cycles before a first BTCC (BTCC1) and after a second BTCC (BTCC2) may be represented by constant functions and baseline threshold values for cycles between BTCC1 and BTCC2 may be represented by a first order function (see Fig. 9, panel (d)) or a second order function or other functions connecting the constant functions (see Fig. 9, panel (f)).

The BT graphs before and after BTCC may be discontinuous with respect to the BTCC (see Fig. 9, panels (e) and (h)). In this case, values of functions for cycles before and after the BTCC with are different from each other when the BTCC is input to each of the functions.

According to an embodiment, the amplification cycles are classified into at least two different groups in terms of at least one baseline threshold-changed cycle (BTCC). Cycles classified into a group is continuous, and have the same baseline threshold value, Cycles classified into immediately adjacent-different groups have different baseline threshold values from each other. Therefore, cycles classified into distantly different groups have different or same baseline threshold values. The BTCC may have the same baseline threshold value as that for cycles before or after the BTCC. The BTCC may be established in the number of one for a data set such that cycles of the data set may be classified into two groups. Alternatively, the BTCC may be established in the number of not less than two for a data set such that cycles of the data set may be classified into not less than three groups.

According to an embodiment, the amplification cycles are classified into at least two different groups in terms of at least one baseline threshold-changed cycle (BTCC) and cycles classified into the same group have the same baseline threshold value. A higher or lower baseline threshold value may be applied to a cycle range showing severe non-specific or noise signals such that non-specific or noise signals are not detected as normal signals. Furthermore, a general baseline threshold value may be applied to the other cycle ranges for detecting and analyzing normal signals.

More particularly, the amplification cycles are classified into two different groups in terms of at least a baseline threshold-changed cycle (BTCC) and cycles classified into the same group have the same baseline threshold value and classified into different groups have different baseline threshold values. The BTCC may have the same baseline threshold value as that for cycles before or after the BTCC.

According to an example, the VBT method is used for amplification results with initial cycles showing abnormal higher slope values. After the BTCC is established, a high baseline threshold value is applied to cycles before the BTCC and low baseline threshold value is applied to cycles after the BTCC for correcting an amplification curve. The application of the VBT method can provide more accurate correction of amplification curves.

The BTCC may be varied depending on patterns of a background or noise signal which are influenced by characteristics of measurement devices, samples and reagents. The BTCC is not limited to a certain cycle range so long as faults due to a background or noise signal can be prevented. For example, the BTCC may be determined with cycles not more than 70, 60, 50, 40, 30, 29, 38, 27, 26, 25, 24, 23, 22, 21, 20 or 15. The BTCC cycles may be determined with cycles not less than 1, 2, 3, 4, 5, 6, 7, 8, 9, 10, 11, 12, 13, 14, 15, 16, 17, 18, 19, 20, 25, 30, 35 or 40. The BTCC may be determined from cycles 1 to 70, particularly cycles 1-60, 1-50, 1-40, 1-30, 5-60, 5-50, 5-40, 10-40, 10-35, 15-35, 15-30, 15-25, more particularly cycles 15-25.

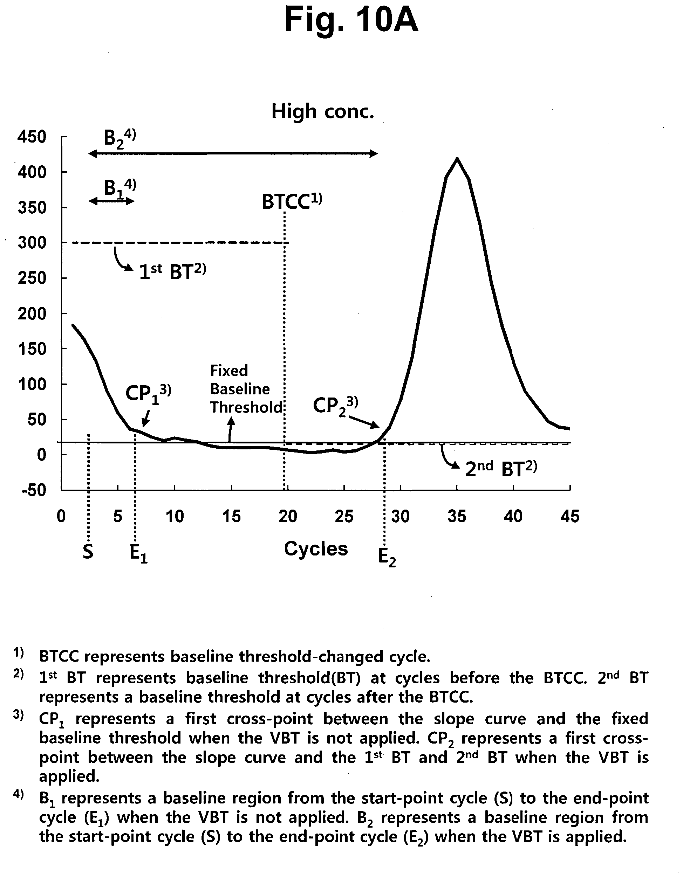

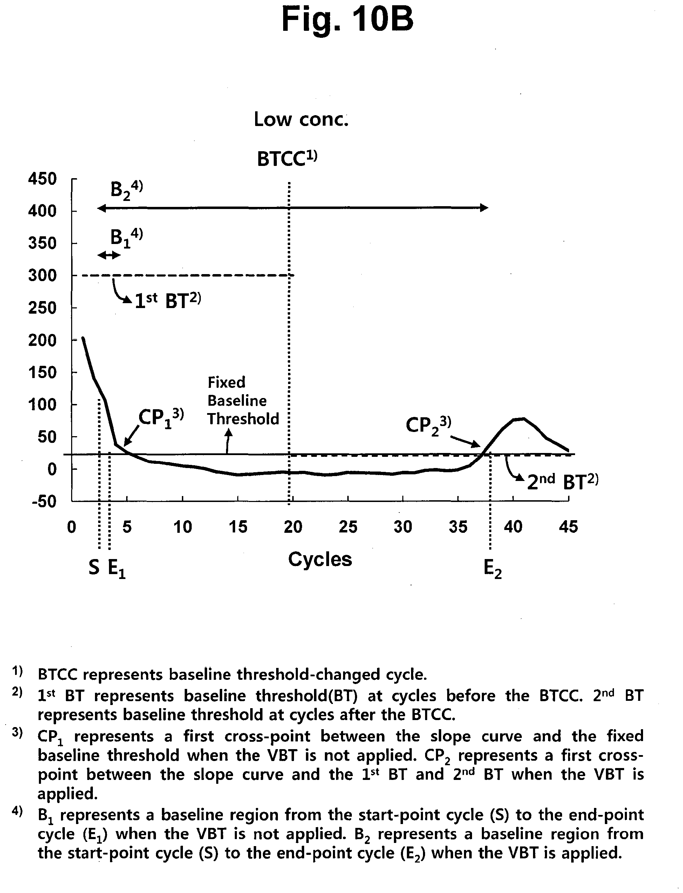

As illustrated in Figs. 10a and 10b, when two functions with respect to the BTCC representing a first BT graph($1^{st}$ BT) and a second BT graph($2^{nd}$ BT) are constant functions, a first cross-point cycle with a slope curve may be determined as the end-point cycle (E2) of a baseline region.

The MBEC method and VBT method may be optionally applied. In addition, both of the two methods may be applied in correction of an amplification curve.

In Example 2, a nucleic acid sample from Influenza A virus (Flu A) is obtained and amplified to provide a first amplification curve. The end-point cycle of a baseline region is determined by applying either the MBEC method or VBT method, or not applying the methods. Afterwards, the first amplification curve is corrected by using a baseline region with the determined end-point cycle. It is found that the corrections by applying either the MBEC method or VBT method can provide corrected amplification curves more accurately reflecting the amount of amplicons compared with corrections not using methods.

*(3) Approach to multiple baseline threshold set*

According to an embodiment, an additional baseline threshold value is applied to at least one cycle among the cycles.

According to an embodiment, an additional baseline threshold set is applied to a data set.

In normal nucleic acid amplification, no existence of target nucleic acid molecules results in an amplification curve with flat shape or a little increased pattern due to non-specific binding and amplification. Unlikely, abnormal negative amplification reaction exhibits decreased values of signal over cycles (*i.e.,* negative slope pattern).

In such case, erroneous baseline region determination and raw data correction are very likely to occur because a cross-point between a baseline threshold set and an amplification curve (or slope curve) is not produced and therefore the end point cycle of a baseline region cannot be established. The application of the additional baseline threshold set by the present invention may prevent such an erroneous analysis. For instance, a baseline threshold value of the additional baseline threshold set may be applied in the negative number and thus enables to determine the end-point cycle of a baseline region having a negative slope value.

According to an embodiment, at least two threshold set are applied to a data set. The approach in which at least two baseline threshold sets are applied to a data set for preventing errors in amplification curve analysis is named herein as "multiple baseline threshold set" method. A baseline threshold set refers to a set of baseline threshold points. The baseline threshold point refers to a coordinate value comprising a cycle and a baseline threshold at the cycle.

According to an embodiment, a baseline threshold set is a variable-baseline threshold set in which baseline threshold values applied to at least two cycles among the cycles are different from each other.

According to an embodiment, a baseline threshold set is a fixed-baseline threshold set in which baseline threshold values applied to the cycles have the same one.

According to an embodiment, multiple baseline threshold sets comprise at least two baseline threshold sets selected from the group consisting of variable-baseline threshold sets and fixed-baseline threshold sets

According to an embodiment, multiple baseline threshold sets comprise at least one fixed-baseline threshold set.

According to an embodiment, multiple baseline threshold sets comprise at least one variable-baseline threshold set.

According to an embodiment, multiple baseline threshold sets comprise at least one fixed-baseline threshold set and at least one variable-baseline threshold set.

According to an embodiment, multiple baseline threshold sets comprise at least two baseline threshold sets, and the both baseline threshold sets are fixed-baseline threshold set.

According to an embodiment, each of multiple baseline threshold sets has a corresponding threshold criterion.

According to an embodiment, multiple baseline threshold sets are simultaneously applied for analyzing a data set. When a plurality of cycles satisfying a threshold criterion are observed, all or a portion of them may be used for analysis.

According to an embodiment, multiple baseline threshold sets are sequentially applied for analyzing a data set. When cycles satisfying a threshold criterion of a firstly applied baseline set are not observed, another baseline set is then applied.

According to an embodiment, a baseline threshold set is applied to a data set and an additional baseline threshold value is applied to at least one cycle among the cycles.

According to an embodiment, the end-point cycle of the baseline region is determined by a process comprising:

(a) applying a baseline threshold value to each of the cycles;
(b) applying one or more additional baseline threshold value to at least one cycle;
(c) identifying one or more cycles satisfying a baseline threshold criterion determined by each of the baseline threshold values; and
(d) determining the end-point cycle of the baseline region with the identified cycle or cycles in the step (c).

The baseline threshold value in the step (a) is applied for end-point determination of normal amplification result.

The additional baseline threshold value in the step (b) is applied for abnormal amplification result, where are no cycles satisfying a main threshold criterion determined by the baseline threshold value in step (a).

More particularly, the cycles satisfying a threshold criterion are identified as follow: when the sign of the subtraction result at cycle n is different from the sign of the subtraction result at cycle (n-1), the cycle n satisfied the threshold criterion, wherein the subtraction result is the result of subtraction the threshold value from the value of signal.

For determination of the end-point cycle, various data sets may be used. Suitable threshold value and determination method may be selected depending on the type of data sets. Those skilled in the art may utilize the present method for analyzing various data sets based on guidance and direction of the embodiments and examples of the slope data set described above.

### Step (c): Establishing Function for Best-Fit Line(S130)

Following determination of the baseline region, a function for a best-fit line of the baseline region is established using at least two data points of the raw data set within the baseline region.

The function for the best-fit line refers to a function best representing inclination shown in data points. The best-fit line refers a graph obtained by plotting the function for the best-fit line.

The function for the best-fit line may be established using at least two data points within the baseline region, for example, a portion or all of data points within the baseline region.

The function for the best-fit line may be established by various approaches, for example, a linear regression analysis or LMS (least mean square) algorithm using data points within the baseline region.

In particular, the function for the best-fit line represented by a first order equation of a linear regression line, "y = mx+b" may be established by using data points from the start-point cycle to the end-point cycle of the baseline region.

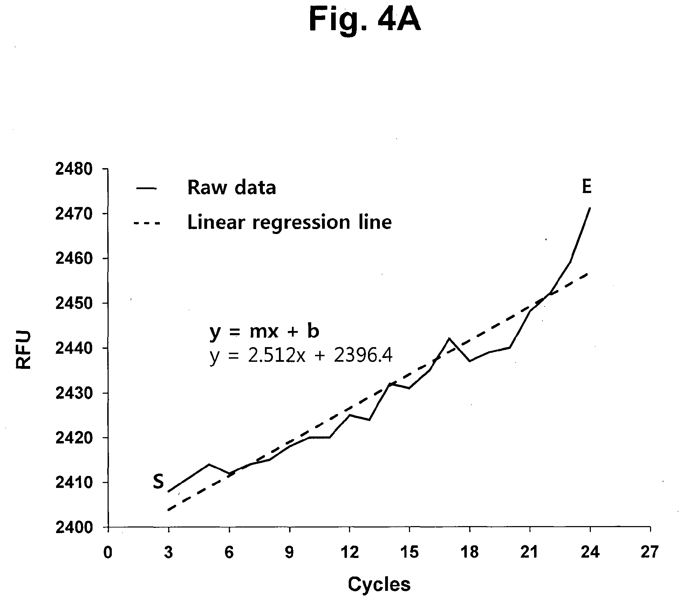

As illustrated in Fig. 4a, "m" as a slope of the best-fit line and "b" is y-intercept of the best-fit line may be calculated by the following mathematical equations 2 and 3:

## Equation 2

$$m = \frac{\sum_{i=S}^{E}(x_i - \bar{x})(y_i - \bar{y})}{\sum_{i=S}^{E}(x_i - \bar{x})^2}$$

## Equation 3

$$b = \frac{\sum_{i=S}^{E}(y_i - mx_i)}{n}$$

$$\text{wherein} \quad \bar{x} = \frac{\sum_{i=S}^{E}x_i}{n} \quad , \quad \bar{y} = \frac{\sum_{i=S}^{E}y_i}{n}$$

m is a slope of the best-fit line, b is y-intercept, $x_i$ is a cycle of $i^{th}$ cycle, $y_i$ is a signal value measured at $i^{th}$ cycle, S is the start-point cycle, E is the end-point cycle of the baseline region, and n is E-S+1.

### Step (d): Obtaining Corrected Data Set(S140)

The corrected data set is obtained by subtracting values of the function for the best-fit line from the values of the signals of the raw data set. The corrected data set contains (i) the amplification cycles of the amplification reaction and (ii) the resultants of the subtraction.

According to an embodiment, the step (a) further comprises plotting the raw data set to provide a first amplification curve and the step (d) further comprises plotting the corrected data set to provide a corrected amplification curve.

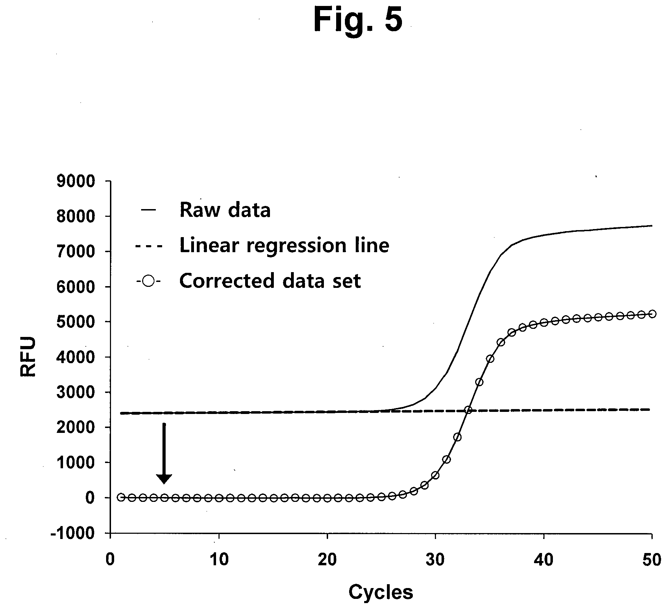

The corrected amplification curve (a second amplification curve) may be obtained by subtracting the best-line from the pre-corrected amplification curve (a first amplification curve) of the raw data set. As illustrated in Fig. 5, the values of the signals of the raw data set are subtracted by values calculated by the function for the best-fit line to obtain the corrected data set and the corrected amplification curve.

According to an embodiment, the correction of the raw data set includes subtracting values of the function for the best-fit line from values of other regions than the baseline region as well as the baseline region. For example, the raw data set is obtained from all cycles and the values of the signals of the raw data set is subtracted by values of the function for the best-fit line to obtain the corrected data set, followed by plotting the corrected data set to provide a corrected amplification curve. Alternatively, a raw data set is obtained from cycles to be included in a baseline region, a function for a best-fit line and a corrected data set are then obtained, and for the other cycles a raw data set is obtained for each signal generation at a cycle and then a corrected data set is obtained by subtracting values of a function for a best-fit line for the corresponding cycle from the value of the signal of the raw data set, followed by plotting all of the corrected data sets to provide a corrected amplification curve.

### III. Storage Medium, Device and Computer Program

Since the storage medium, the device and the computer program of the prevent invention described hereinbelow are intended to perform the present methods in a computer, the common descriptions between them are omitted in order to avoid undue redundancy leading to the complexity of this specification.

In still another aspect of this invention, there is provided a A computer readable storage medium containing instructions to configure a processor to perform a method for analyzing a sample, the method comprising:

(a) receiving a value of signal at each of cycles of a signal-generating process using the sample to provide values of signals at the cycles;
(b) applying a threshold value to each of the cycles such that a plurality of threshold values are applied to the cycles; wherein the threshold values of at least two cycles among the cycles are different from each other;
(c) identifying one or more cycles satisfying a threshold criterion determined by each of the threshold values; and
(d) analyzing the sample by using the identified cycle or cycles in the step (c).

According to an embodiment, the signal-generating process generates signal in a dependent manner on the presence of an analyte in the sample; wherein the analyte is a target nucleic acid molecule; wherein the signal-generating process is a process with amplification or with no amplification of the target nucleic acid molecule; wherein the process with amplification of the target nucleic acid molecule is real-time polymerase chain reaction (real-time PCR).

According to an embodiment, the signal-generating process is real-time PCR, the value of signals are mathematically modified values of signals generated from the real-time PCR, and the mathematically modified values are obtained by differentiating the values of signals with respect to the cycles.

According to an embodiment, the threshold values are determined in such a manner that with respect to a threshold-changed cycle (TCC), a function formed by a set of pre-TCC cycles and threshold values to be applied to the pre-TCC cycles is different from a function formed by a set of post-TCC cycles and threshold values to be applied to the post-TCC cycles.

In further aspect of this invention, there is provided a computer readable storage medium containing instructions to configure a processor to perform a method for correcting a raw data set of an amplification reaction using a signal-generating means, the method comprising:

(a) receiving the raw data set containing (i) amplification cycles of the amplification reaction and (ii) values of signals obtained from the signal-generating means at the amplification cycles;
(b) determining a baseline region by determining both a start-point cycle and an end-point cycle of the baseline region using the raw data set;
(c) establishing a function for a best-fit line of the baseline region using at least two data points of the raw data set within the baseline region; and
(d) obtaining a corrected data set by subtracting values of the function for the best-fit line from the values of the signals of the raw data set; wherein the corrected data set contains (i) the amplification cycles of the amplification reaction and (ii) the resultants of the subtraction.

According to an embodiment, the end-point cycle of the baseline region in the step (b) is determined by a process comprising:
(b1) applying a baseline threshold value to each of the amplification cycles such that a plurality of baseline threshold values are applied to the cycles; wherein at least two cycles among the cycles have different baseline threshold values from each other; (b2) identifying one or more cycles satisfying a baseline threshold criterion determined by each of the baseline threshold values; (b3) determining the end-point cycle of the baseline region by using the identified cycle or cycles in the step (b2).

According to an embodiment, the slope in the step (b1) is a slope calculated by a least square method using a data point of a certain cycle and at least one data point of a cycle or cycles before and/or after the certain cycle.

In still another aspect of this invention, there is provided a computer program to be stored on a computer readable storage medium to configure a processor to perform a method for analyzing a sample, the method comprising:

(a) receiving a value of signal at each of cycles of a signal-generating process using the sample to provide values of signals at the cycles;
(b) applying a threshold value to each of the cycles such that a plurality of threshold values are applied to the cycles; wherein the threshold values of at least two cycles among the cycles are different from each other;
(c) identifying one or more cycles satisfying a threshold criterion determined by each of the threshold values; and
(d) analyzing the sample by using the identified cycle or cycles in the step (c).

According to an embodiment, there is provided a computer program stored on a computer readable storage medium to configure a processor to perform the method for analyzing a sample.

According to an embodiment, the signal-generating process generates signal in a dependent manner on the presence of an analyte in the sample; wherein the analyte is a target nucleic acid molecule; wherein the signal-generating process is a process with amplification or with no amplification of the target nucleic acid molecule; wherein the process with amplification of the target nucleic acid molecule is real-time polymerase chain reaction (real-time PCR).

According to an embodiment, the signal-generating process is real-time PCR, the value of signals are mathematically modified values of signals generated from the real-time PCR, and the mathematically modified values are obtained by differentiating the values of signals with respect to the cycles.

According to an embodiment, the threshold values are determined in such a manner that with respect to a threshold-changed cycle (TCC), a function formed by a set of pre-TCC cycles and threshold values to be applied to the pre-TCC cycles is different from a function formed by a set of post-TCC cycles and threshold values to be applied to the post-TCC cycles.

In further aspect of this invention, there is provided a computer program to be stored on a computer readable storage medium to configure a processor to perform a method for correcting a raw data set of an amplification reaction using a signal-generating means, the method comprising:

(a) receiving the raw data set containing (i) amplification cycles of the amplification reaction and (ii) values of signals obtained from the signal-generating means at the amplification cycles;
(b) determining a baseline region by determining both a start-point cycle and an end-point cycle of the baseline region using the raw data set;
(c) establishing a function for a best-fit line of the baseline region using at least two data points of the raw data set within the baseline region; and
(d) obtaining a corrected data set by subtracting values of the function for the best-fit line from the values of the signals of the raw data set; wherein the corrected data set contains (i) the amplification cycles of the amplification reaction and (ii) the resultants of the subtraction.

According to an embodiment, there is provided a computer program stored on a computer readable storage medium to configure a processor to perform the method for correcting a raw data set of an amplification reaction using a signal-generating means.

According to an embodiment, the end-point cycle of the baseline region in the step (b) is determined by a process comprising:

(b1) applying a baseline threshold value to each of the amplification cycles such that a plurality of baseline threshold values are applied to the cycles; wherein at least two cycles among the cycles have different baseline threshold values from each other; (b2) identifying one or more cycles satisfying a baseline threshold criterion determined by each of the baseline threshold values; (b3) determining the end-point cycle of the baseline region by using the identified cycle or cycles in the step (b2).

According to an embodiment, the slope in the step (b1) is a slope calculated by a least square method using a data point of a certain cycle and at least one data point of a cycle or cycles before and/or after the certain cycle.

The program instructions are operative, when preformed by the processor, to cause the processor to perform the present method described above. The program instructions for performing the method for analyzing a sample may comprise an instruction to receive a value of signal at each of cycles of a signal-generating process using the sample to provide values of signals at the cycles; an instruction to apply a threshold value to each of the cycles and identify one or more cycles satisfying a threshold criterion determined by each of the threshold values; and an instruction to analyze the sample by using the identified cycle or cycles. The program instructions for performing the method for correcting a raw data set of an amplification reaction comprise an instruction to receive the raw data set; an instruction to determine a baseline region by determining both a start-point cycle and an end-point cycle of the baseline region and establish a function for a best-fit line of the baseline region; and an instruction to obtain a corrected data set by subtracting values of the function for the best-fit line from the values of the signals of the raw data set.

The present method described above is implemented in a processor, such as a processor in a stand-alone computer, a network attached computer or a data acquisition device such as a real-time PCR machine.

The types of the computer readable storage medium include various storage medium such as CD-R, CD-ROM, DVD, flash memory, floppy disk, hard drive, portable HDD, USB, magnetic tape, MINIDISC, nonvolatile memory card, EEPROM, optical disk, optical storage medium, RAM, ROM, system memory and web server.

The data points (*e.g.,* signal intensity and amplification cycles) may be received through several mechanisms. For example, the data points may be acquired by a processor resident in a PCR data acquiring device. The data points may be provided to the processor in real time as the data points are being collected, or it may be stored in a memory unit or buffer and provided to the processor after the experiment has been completed. Similarly, the data set may be provided to a separate system such as a desktop computer system via a network connection (*e.g.,* LAN, VPN, intranet and Internet) or direct connection (*e.g.,* USB or other direct wired or wireless connection) to the acquiring device, or provided on a portable medium such as a CD, DVD, floppy disk, portable HDD or the like to a stand-alone computer system. Similarly, the data set may be provided to a server system via a network connection (*e.g.,* LAN, VPN, intranet, Internet and wireless communication network) to a client such as a notebook or a desktop computer system.

After the data points have been received or acquired, the data analysis process proceeds to analyze a sample or obtain a corrected data set of an amplification reaction. For example, the processor for analyzing a sample processes the received data points to identify one or more cycles satisfying a threshold criterion determined by each of the threshold values. The processor for obtaining a corrected data set of an amplification reaction processes the received data points to determine a baseline region, establish a function for a best-fit line of the baseline region and obtain a corrected data set by subtracting values of the function for the best-fit line from the values of the signals of the raw data set.

The instructions to configure the processor to perform the present invention may be included in a logic system. The instructions may be downloaded and stored in a memory module (*e.g.,* hard drive or other memory such as a local or attached RAM or ROM), although the instructions can be provided on any software storage medium such as a portable HDD, USB, floppy disk, CD and DVD. A computer code for implementing the present invention may be implemented in a variety of coding languages such as C, C++, Java, Visual Basic, VBScript, JavaScript, Perl and XML. In addition, a variety of languages and protocols may be used in external and internal storage and transmission of data and commands according to the present invention.

In still further aspect of this invention, there is provided a device for analyzing a sample, comprising (a) a computer processor and (b) the computer readable storage medium described above coupled to the computer processor.

In another aspect of this invention, there is provided a device for correcting a raw data set of an amplification reaction using a signal-generating means, comprising (a) a computer processor and (b) the computer readable storage medium described above coupled to the computer processor.

According to an embodiment, the device further comprises a reaction vessel to accommodate the sample and signal-generating means, a temperature controlling means to control temperatures of the reaction vessel and/or a detector to detect signals at amplification cycles.

According to an embodiment, the computer processor permits not only to receive values of signals at cycles but also to analyze a sample or obtain a corrected data set of an amplification reaction. The processor may be prepared in such a manner that a single processor can do two performances: direction of receiving data points and analyzing a sample or obtaining a corrected data set. Alternatively, the processor unit may be prepared in such a manner that two processors do two performances, respectively.

According to an embodiment, the processor may be embodied by installing software into conventional devices for detection of target nucleic acid sequences (*e.g.* real-time PCR device).

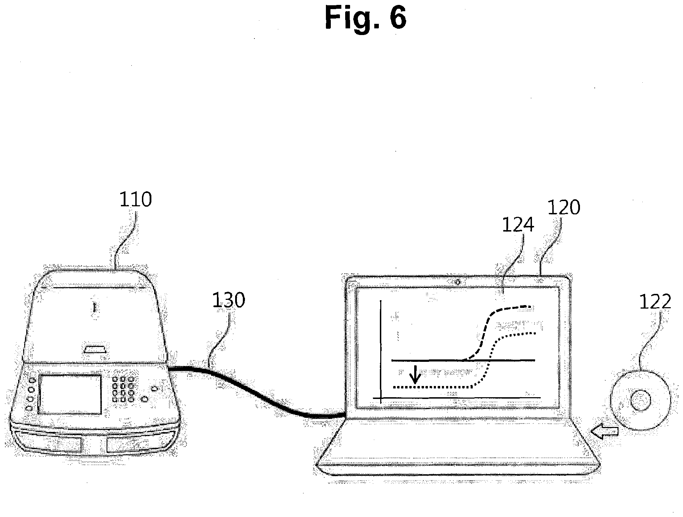

Fig. 6 illustrates a real-time PCR system implementing an embodiment of the present invention for correcting a raw data set of an amplification reaction. The system comprises a real-time PCR device (110) for performing a real-time PCR amplification, and a computer system (120) as a logic system connected to the real-time PCR device (110) via a cable (130) for correcting the raw data set and displaying the correction resultants. The computer system (120) may display the correction resultants in various forms such as graphs, tables and words according to demands of users. The computer system (120) may comprise instructions contained in a computer readable storage medium for performing the present method for correcting an amplification curve of an amplification reaction. The real-time PCR device (110) and the computer system (120) may be integrated into a system.

Data points (*e.g.,* signal intensities and amplification cycles) associated with amplification curves may be received in various fashions. For example, data points may be received and collected by a processor in a data collector of the real-time PCR device (110). Upon collecting the data points, they may be provided to a processor in a real-time manner, or stored in a memory unit or buffer and then provide to a processor after experiments.

Likely, the data set may be provided from the real-time PCR device (110) to the computer system (120) such as a desktop computer system via network connection (*e.g.,* LAN, VPN, intranet and internet) or direct connection (*e.g.,* USB and wired or wireless direct connections), or via portable media such as CD, DVD, floppy disk and portable HDD. Alternatively, the data set may be provided to a server system via network connections (*e.g.,* LAN, VPN, intranet, internet and wireless communication network) connected to a client such as notebook and desktop computer systems.

After the data set is received or obtained, a data analysis processor proceeds to provide a data set reflecting a corrected amplification curve.

The correction of amplification curves may be undertaken by an application (*i.e.,* program) installed into the computer system (120). Alternatively, the correction of amplification curves may be made by an application directly installed into the computer system (120) through application store server or application provider servers in which the application is operable in an operating system of the computer system (120). The operating system includes Window, Macintosh and mobile operating systems such as iOS and Android that are installed into mobile terminals such as Smartphones and Tablet PC.

As described above, the present method for correcting amplification curves may be embodied by an application (*i.e.,* program) supplier-installed or user-direct installed into the computer system (120), and recorded in a computer readable storage medium (122).

A computer program (124) embodying the present method for correcting amplification curves may implement all functions for the correction. The computer program (124) may a program comprising program instructions stored on a computer readable storage medium to configure a processor to perform the present method.

The computer program (124) may be coded by using suitable computer languages such as C, C++, JAVA, Visual basic, VBScript, JavaScript, Perl, XML and machine languages. The program codes may include function codes for mathematical functions described above and control codes for implementing process in order by a processor of the computer system (120).

The codes may further comprise memory reference codes by which additional information or media required in implementing the above-described functions by the processor is referred at location (address) of internal or external memory of the computer system (120).

When the computer system (120) requires communication with another computer or server in remote for implementing functions of the processor, the codes may further comprise communication-relating codes encoding how the processor is communicated with another computer or server in remote by using communication module (e.g., wired and/or wireless communication module) or what information or media is transmitted.

Functional programs and codes (code segments) for embodying the present invention may be easily inferred or modified by programmers in the art in considering system environments of computers reading storage media and executing programs.

The storage medium (122) network-connected to the computer system (120) may be distributed and computer-readable codes may be stored and executed in a distribution manner. In such case, at least one computer among a plurality of distributed computers may implement a portion of the functions and transmit results of the implementation to at least one computer that may also implement a portion of the functions and transmit results of the implementation to at least one computer.

The storage medium (122) in which application (*i.e.,* program) is recorded for executing the present invention includes a storage medium (*e.g.,* hard disk) contained in application store servers or application provider servers, application provider servers *per se,* another computer having the program and its storage medium.

The computer system (120) capable of reading the storage medium (122) may include general PC such as desk top or notebook computers, mobile terminals such as Smartphone, Tablet PC, PDA (Personal Digital Assistants) and mobile communication terminals as well as all computing-executable devices.

The features and advantages of this invention will be summarized as follows:

(a) The present invention for analyzing a sample prevents from determining cycles based on false signals usually observed in a multitude of reactions and processes, thereby much more accurately obtaining information for analyzing a sample.

(b) In the present invention for analyzing a sample, a threshold value is applied to each of the cycles such that a plurality of threshold values are applied to the cycles in a distinct manner, thereby eliminating influence of abnormal signals on analysis of the sample. Conventional technologies eliminate abnormal signals in analysis of the sample by analyzing signals *per se.* Therefore, the present method may be executed by using different algorithm from those for conventional technologies and therefore may be used along with the conventional technologies, which dramatically enhances accuracy of sample analysis.

(c) As the present invention permits to correct an amplification curve by establishing a more-accurate baseline region for each sample (or PCR reaction), results of amplification reactions may be analyzed more accurately and reliably.

(d) As the present invention corrects amplification curves by a concise process (or algorithm), its optimization depending on subjects to be analyzed and devices for measurement may be much easier.

The present invention will now be described in further detail by examples. It would be obvious to those skilled in the art that these examples are intended to be more concretely illustrative and the scope of the present invention as set forth in the appended claims is not limited to or by the examples.

## EXAMPLES

### EXAMPLE 1: Correction of Amplification Curves (I)

Using a real-time PCR system shown in Fig. 6, we examined whether the amplification curve is corrected by the best fit line of a baseline region derived from slope curve of the amplification curve and a baseline threshold as follows.

### Preparation of Raw Data Set (Pre-Corrected Amplification Curve) (S110)

*Taq* DNA polymerase having a 5' nuclease activity was used for the extension of upstream primers and downstream primers and the cleavage of a TaqMan probe. Genomic DNA of *Neisseria gonorrhoeae* (NG) were used as target nucleic acid sequences.

TaqMan real-time PCR was employed to detect NG. If target nucleic acid is present, a TaqMan probe is cleaved and a labeled fragment is released. An amplification curve can be obtained by measuring a signal from the labeled fragment.

A TaqMan probe for NG is labeled with a fluorescent reporter molecule (Cal Fluor Red 610) at its 5'-end and a quencher molecule (BHQ-2) at its 3'-end (SEQ ID NO: 3).

The sequences of upstream primer, downstream primer, and probe used in this Example are:

NG-F    5'-TACGCCTGCTACTTTCACGCTIIIIIGTAATCAGATG-3' (SEQ ID NO: 1)
NG-R    5'-CAATGGATCGGTATCACTCGCIIIIICGAGCAAGAAC-3' (SEQ ID NO: 2)
NG-P    5'-[Cal Fluor Red 610]TGCCCCTCATTGGCGTGTTTCG[BHQ-2]-3' (SEQ ID NO: 3)

(I: Deoxyinosine, BHQ-2: Black hole quencher-2)

The real-time PCR was conducted in the final volume of 20 μl containing a target nucleic acid (10 pg, 1 pg, 100 fg, 10 fg, or 1 fg of NG genomic DNA), 5 pmole of upstream primer (SEQ ID NO: 1) and 5 pmole of downstream primer (SEQ ID NO: 2) for NG target amplification, 3 pmole of TaqMan probe (SEQ ID NO: 3), and 5 μl of 4X Master Mix [final, 200 uM dNTPs, 2 mM MgCl$_2$, 2 U of *Taq* DNA polymerase]. The tubes containing the reaction mixture were placed in the real-time thermocycler (CFX96, Bio-Rad) for 5 min at 50°C, denatured for 15 min at 95°C and subjected to 50 cycles of 30 sec at 95°C, 60 sec at 60°C, and 30 sec at 72°C. Detection of a signal was performed at 60°C of each cycle.

A raw data set was obtained by the real time PCR amplification and a pre-corrected amplification curve was plotted by using the raw data set (see Fig. 2).

The pre-corrected amplification curve was corrected as follows:

### Determination of Baseline Region (S120)

The third (3$^{rd}$) cycle of the amplification reaction was determined as a start-point cycle (S) of a baseline region.

For determining an end-point cycle (E) of the baseline region, a slope curve was obtained from the raw data set by linear regression analysis (LRA) using a least square method expressed by Mathematical Equation 1.

The data for the three cycles i-1, i and i+1 were used for calculating slope values of i$^{th}$ cycle (i.e. a=1 and b=1).

In order to avoid crossing with background signals at initial cycles before producing peaks in a slope curve, a baseline threshold value was determined as "20". A first cross-point (CP) cycle between the baseline threshold and the slope curve was determined as the end-point cycle (E). Afterwards, the baseline region was finally determined (see Fig. 3).

### Obtaining Function for Best Fit Line in Baseline Region (S130)

By using data at the cycles from the start-point cycle (S) to the end-point cycle (E) in the baseline region determined above, a least square method was undertaken to obtain a best fit line in the form of a linear equation of a linear regression line (see Fig. 4a).

The general linear equation of the linear regression line is "y=mx+b" in which "m" as a slope was calculated by Mathematical Equation 2 and "b" as y-intercept was calculated by Mathematical Equation 3. Then, the function for the best fit line in the form of the linear equation of the linear regression line is "y=2.512x+2396.4".

### Obtaining Corrected Data Set (Corrected Amplification Curve) (S140)

A corrected data set was obtained by subtracting the pre-corrected amplification curve for 1-50 cycles by the best fit line obtained above and plotted for obtaining a corrected amplification curve.

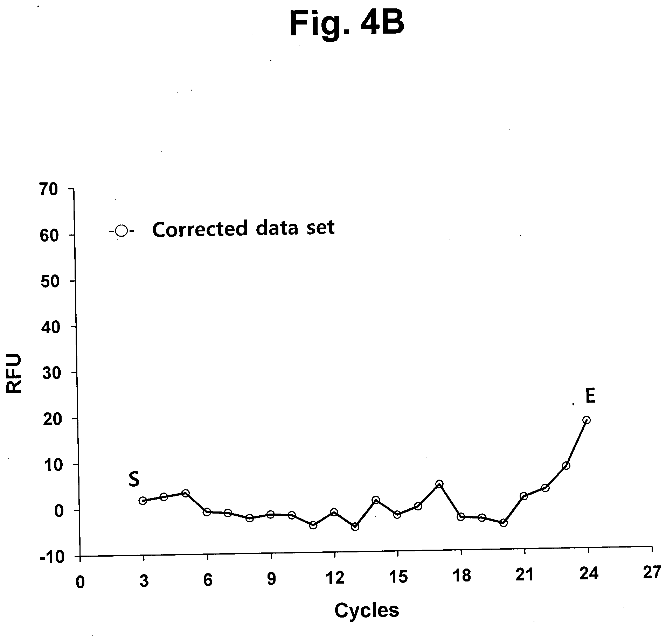

Fig. 4b represents the corrected data set obtained by subtracting the raw data set for cycles in the baseline region of Fig. 2 by data of the function for the best fit line in the baseline region.

Fig. 5 represents the corrected amplification curve obtained by plotting the corrected data set.

As the correction of amplification curves can be made in accordance with the present invention using uncomplicated algorithms, the present invention can optimize particular conditions for measured samples and measurement devices in much easier manner.

## EXAMPLE 2: Correction of Amplification Curves (II)

We examined whether errors in determination of a baseline region for correcting amplification curves obtained in a real-time PCR may be removed.

*Taq* DNA polymerase having a 5' nuclease activity was used for the extension of upstream primers and downstream primers and the cleavage of a TaqMan probe. Genomic RNA of Influenza A virus (Flu A) was used as target nucleic acid sequences.

TaqMan real-time PCR was employed to detect Flu A. If target nucleic acid is present, a TaqMan probe is cleaved and a labeled fragment is released. An amplification curve can be obtained by measuring a signal from the labeled fragment.

A TaqMan probe for Flu A is labeled with a fluorescent reporter molecule (FAM) at its 5'-end and a quencher molecule (BHQ-1) at its 3'-end (SEQ ID NO: 6).

The sequences of upstream primer, downstream primer, and probe used in this Example are:

> Flu A-F  5'- TGGAATGGCTAAAGACAAGACCIIIIITGTCACCTCT-3' (SEQ ID NO: 4)
> Flu A-R  5'-CATCCTGTTGTATATGAGGCCCATIIIICTGGCAAG-3' (SEQ ID NO: 5)
> Flu A-P  5'-[FAM] CTCACTGGGCACGGTGAGCGTGA [BHQ-1]-3' (SEQ ID NO: 6)

(I: Deoxyinosine, BHQ-1: Black hole quencher-1)

The real-time PCR was conducted in the final volume of 25 $\mu$l containing a target nucleic acid ($10^{-3}$, $10^{-4}$, $10^{-5}$, or $10^{-6}$ dilution of the extracted Flu A genomic RNA), 5 pmole of upstream primer (SEQ ID NO: 4) and 5 pmole of downstream primer (SEQ ID NO: 5) for Flu A target amplification, 3 pmole of TaqMan probe (SEQ ID NO: 6), 5 $\mu$l of 5X RT-PCR buffer [75 mM Tris-HCl (pH 8.3), 50 mM KCl, 2.5 mM MgCl$_2$, 0.2 mM dNTP], and 2 $\mu$l of Enzyme Mix [final, 3.5 U of Taq DNA polymerase, 25 U of MMLV Reverse transcriptase, 5 U of RNase inhibitor]. The tubes containing the reaction mixture were placed in the real-time thermocycler (CFX96, Bio-Rad) for 20 min at 50°C, denatured for 15 min at 95°C and subjected to 45 cycles of 10 sec at 95°C, 60 sec at 60°C, and 10 sec at 72°C. Detection of a signal was performed at 60°C of each cycle.

## Identification of Errors in Determination of Baseline Region (S120)

The end-point cycle (E) of a baseline region may be determined as a cycle at or around which an increase in a real target signal intensity in amplification reactions is initiated.

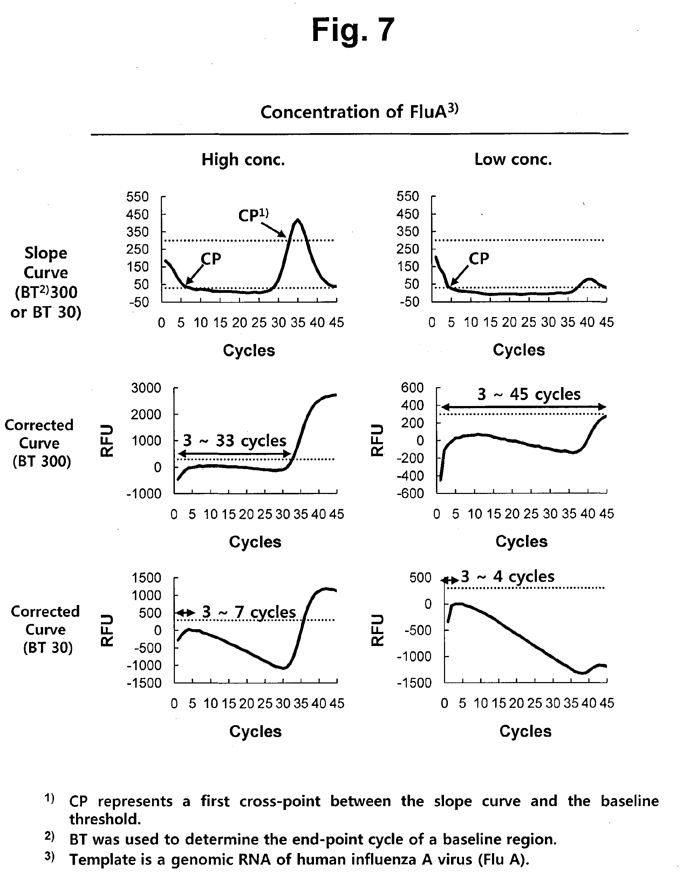

An end-point cycle of a baseline region may be determined in considering both a slope calculated at each cycle and a threshold value at each cycle. In such case, a baseline threshold value may be applied over all amplification cycles as Example 1; however this approach may produce errors in determination of a baseline region (see Fig. 7).

For instance, when the baseline threshold value is established as low as "30", a point of generating an initial noise signal may be determined as the end-point cycle of a baseline region instead of a point of initiating the increase in a real target signal, thereby leading to occurrence of errors in determination of a baseline region. On the other hand, when the baseline threshold value is established as high as "300", a point of initiating the increase in a real target signal may not be detected from a sample containing a target sequence of low concentration (*i.e.,* a sample with lower slope values), thereby leading to occurrence of errors in determination of a baseline region.

As such, it would be understood that a corrected amplification curve not reflecting an actual amount of amplicons may be obtained due to errors in determination of a baseline region.

## Determination of End-point Cycle by MBEC method

The third (3rd) cycle of the amplification reaction was determined as a start-point cycle (S) of a baseline region.

As shown in Figs. 8a and 8b, the end-point cycle in a slope curve can be determined with a cycle after a minimum baseline end-point cycle (MBEC). In Example 2, the tenth (10th) cycle was determined as MBEC.

As shown in Figs. 8a (a high concentration sample) and 8b (a low concentration sample), when MBEC was not adopted, the baseline region ($B_1$) with the end-point cycle as a first cross-point ($CP_1$) between the baseline threshold and the slope curve was determined as Cycles 3-7 (high-conc. sample) or Cycles 3-4 (low-conc. sample). On the other hand, when MBEC was adopted, the baseline region ($B_2$) with the end-point cycle as a first cross-point ($CP_2$) over MBEC was determined as Cycles 3-29 (high-conc. sample) or Cycles 3-38 (low-conc. sample).

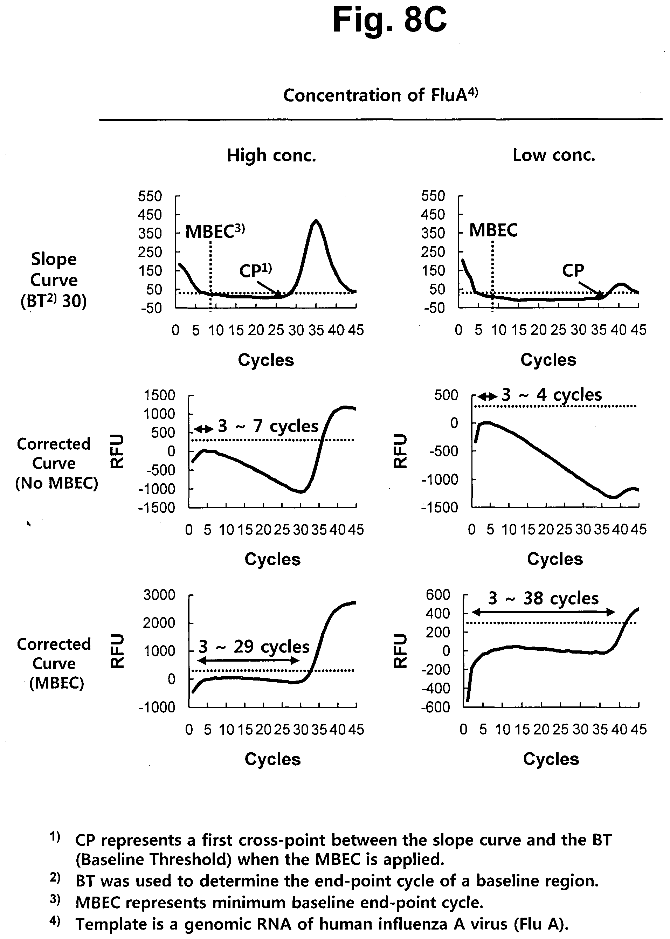

As shown in Fig. 8c, when the baseline threshold was determined as "30" and MBEC was not adopted, the corrected amplification curves were shown to represent inaccurate amounts of amplicons or false negative results. When the baseline threshold was determined as "30" and MBEC was adopted, the corrected amplification curves reflected accurate amounts of amplicons.

As such, a point of generating an initial noise signal may be determined as the end-point cycle of a baseline region due to noise signals frequently found in initial cycles of amplification reactions, thereby leading to occurrence of errors in determination of a baseline region. Those results urge us to reason that errors in determination of a baseline region (S120) can be successfully eliminated by the present invention.

**Determination of End-point Cycle by VBT (Variable Baseline Threshold) method**

VBT method adopted in Example 2 is carried out in such a manner that a baseline threshold-changed cycle (BTCC) is determined and different baseline thresholds are applied to cycles before and after BTCC, respectively.

BTCC was determined as Cycle 20 and a first BT (baseline threshold) and a second BT were differentially applied to Cycles 1-20 and Cycles 21-45, respectively. The first BT was determined as "300" and the second BT as "30".

As shown in Figs. 10a (a high concentration sample) and 10b (a low concentration sample), when VBT was not adopted and the fixed baseline threshold of "30" was applied to all cycles, the end-point cycle ($E_1$) was determined as Cycle 7 for high-concentration sample or Cycle 4 for low-concentration sample.

As the third ($3^{rd}$) cycle of the amplification reaction was determined as a start-point cycle (S) of a baseline region, the baseline region ($B_1$) was determined as Cycles 3-7 (high-conc. sample) or Cycles 3-4 (low-conc. sample).

On the other hand, when VBT was adopted, the end-point cycle ($E_1$) was determined as Cycle 29 for high-concentration sample or Cycle 38 for low-concentration sample. Thus, the baseline region ($B_2$) was determined as Cycles 3-29 (high-conc. sample) or Cycles 3-38 (low-conc. sample).

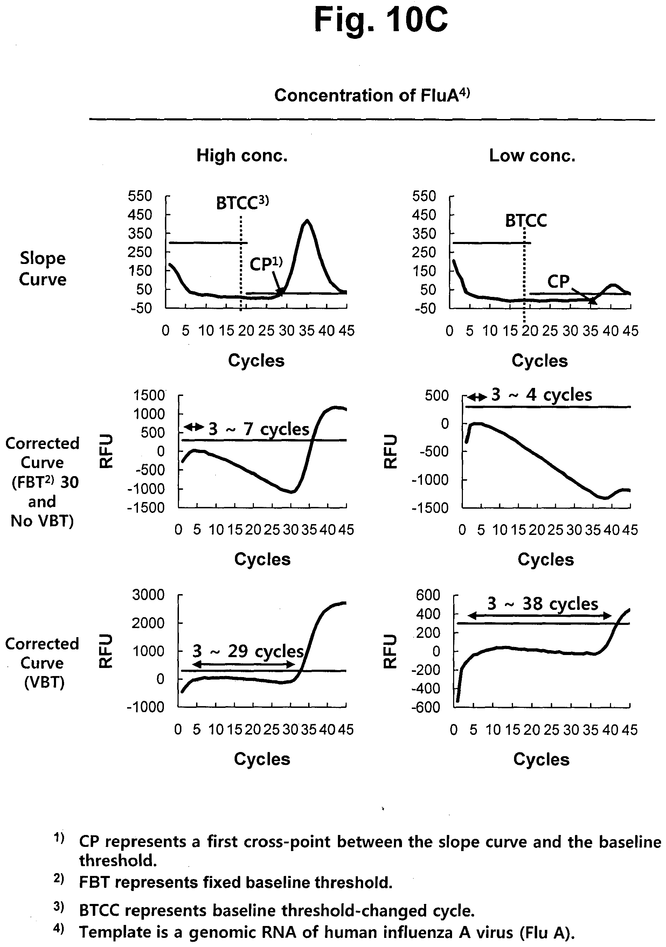

As shown in Fig. 10c, when VBT was not adopted, the corrected amplification curves were shown to represent inaccurate amounts of amplicons or false negative results. When VBT was adopted, the corrected amplification curves reflected accurate amounts of amplicons or no false negative results.

Therefore, it would be appreciated that the VBT method establishing variable baseline thresholds differentially can eliminate errors of misinterpreting a point of generating an initial noise signal in initial amplification cycles as the end-point cycle (E) of a baseline region. Furthermore, the VBT method is capable of determining more accurately an initiating point of signal increase in later amplification cycles, thereby eliminating errors in determination of a baseline region (S120).

According to the results in Example 2, it would be understood that the baseline threshold can be determined with no interference of background signals in early cycles.

As described above, the present invention can analyze amplification results in more reliable and accurate manner by correcting amplification curves through error-free determination of a baseline region.

**EXAMPLE 3: Detection and quantification of target nucleic acid by accurate Ct value determination.**

We examined whether errors in determination of Ct value from amplification curves may be eliminated.

*Taq* DNA polymerase having a 5' nuclease activity was used for the extension of upstream primers and downstream primers and the cleavage of a TaqMan probe. Genomic RNA of Influenza A virus (Flu A) were used as target nucleic acid sequences.

TaqMan real-time PCR was employed to detect Flu A. If target nucleic acid is present, a TaqMan probe is cleaved and a labeled fragment is released. An amplification curve can be obtained by measuring a signal from the labeled fragment.

A TaqMan probe for Flu A is labeled with a fluorescent reporter molecule (FAM) at its 5'-end and a quencher molecule (BHQ-1) at its 3'-end (SEQ ID NO: 6).

The sequences of upstream primer, downstream primer, and probe used in this Example are:

Flu A-F    5'-TGGAATGGCTAAAGACAAGACACCIIIIITGTCACCTCT-3' (SEQ ID NO: 4)
Flu A-R    5'-CATCCTGTTGTATATGAGGCCCATIIIICTGGCAAG-3' (SEQ ID NO: 5)
Flu A-P    5'-[FAM] CTCACTGGGCACGGTGAGCGTGA [BHQ-1]-3' (SEQ ID NO: 6)

(I: Deoxyinosine, BHQ-1: Black hole quencher-1)

The real-time PCR was conducted in the final volume of 25 $\mu$l containing a target nucleic acid ($10^{-3}$, $10^{-4}$, $10^{-5}$, $10^{-6}$, or $10^{-7}$ dilution of the extracted Flu A genomic RNA), 5 pmole of upstream primer (SEQ ID NO: 4) and 5 pmole of downstream primer (SEQ ID NO: 5) for Flu A target amplification, 3 pmole of TaqMan probe (SEQ ID NO: 6), 5 $\mu$l of 5X RT-PCR buffer [75 mM Tris-HCl (pH 8.3), 50 mM KCl, 2.5 mM $MgCl_2$, 0.2 mM dNTP], and 2 $\mu$l of Enzyme Mix [final, 3.5 U of Taq DNA polymerase, 25 U of MMLV Reverse transcriptase, 5 U of RNase inhibitor]. The tubes containing the reaction mixture were placed in the real-time thermocycler (CFX96, Bio-Rad) for 20 min at 50°C, denatured for 15 min at 95°C and subjected to 45 cycles of 10 sec at 95°C, 60 sec at 60°C, and 10 sec at 72°C. Detection of a signal was performed at 60°C of each cycle.

## Identification of Errors in Determination of Ct value

The traditional cycle threshold (Ct) method for obtaining the accurate amount of target nucleic acids from amplification curve typically uses a signal threshold. The Ct value is determined based on the point within the exponential phase of the amplification curve where the fluorescence response increases above the background signal level to cross a predetermined signal threshold value. In such case, using a fixed signal threshold (FST) value may produce errors in determination of the Ct value.

Fig. 11a represents the corrected amplification curve obtained from ($10^{-3}$) dilution of the extracted Flu A genomic RNA. As shown in Fig. 11a, when the FST value is established as low as "200" RFU, a point of generating an initial noise signal may be determined as the Ct value instead of a point of exponentially increasing a real target signal, thereby leading to occurrence of errors in determination of target nucleic acid concentration.

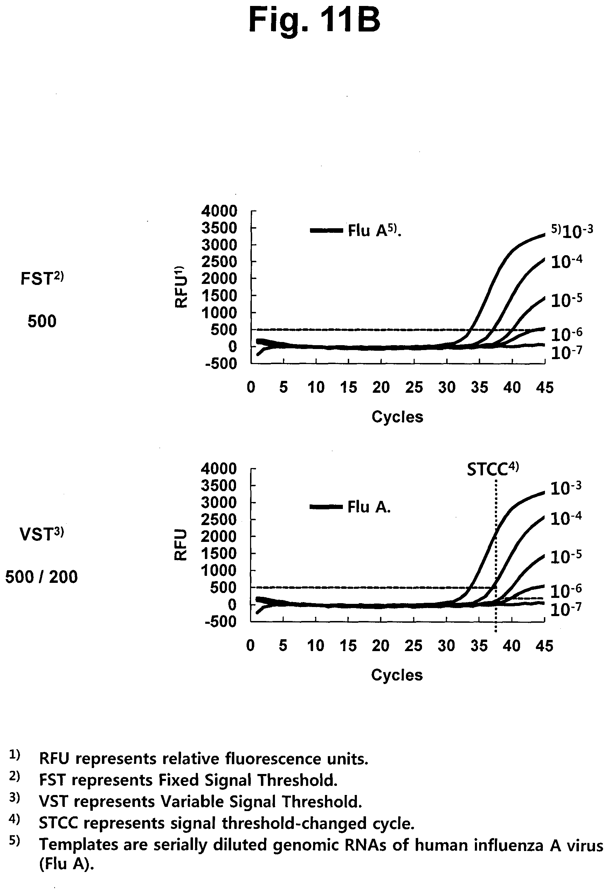

Fig. 11b represents the corrected amplification curves obtained from ($10^{-7} \sim 10^{-3}$) dilutions of the extracted Flu A genomic RNA. As shown in Fig. 11b, when the FST value is established as high as "500" RFU, a sample containing a target sequence of low concentration may be determined as amount less than the actual amount of target nucleic acids.

As such, it would be appreciated that the traditional Ct method establishing a fixed signal threshold cannot eliminate errors in determination of target nucleic acid concentration.

## Determination of Ct value by VST (Variable Signal Threshold) method

VST method adopted in Example 3 is carried out in such a manner that a signal threshold-changed cycle (STCC) is determined and different signal thresholds are applied to cycles before and after STCC, respectively.

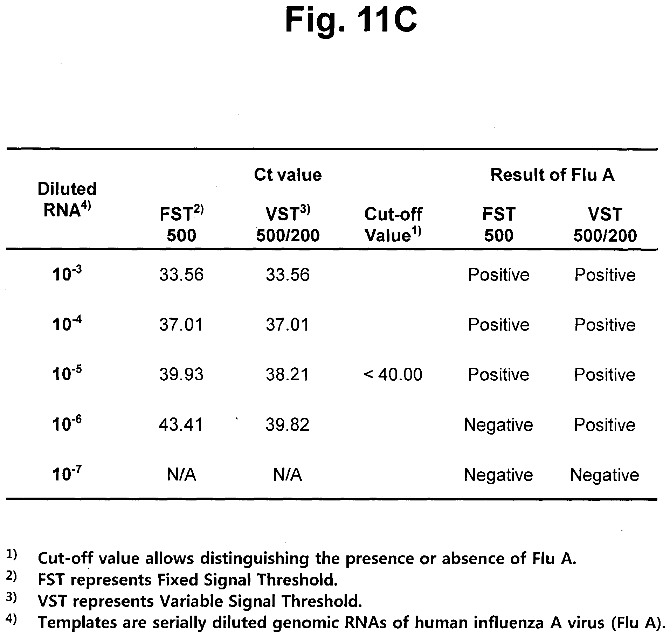

In Fig. 11a, STCC was determined as Cycle 10 and a first ST (signal threshold) and a second ST were differentially applied to Cycles 1-10 and Cycles 11-45, respectively. In Figs. 11b and 11c, STCC was determined as Cycle 38 and a first ST and a second ST were differentially applied to Cycles 1-38 and Cycles 39-45, respectively. The first ST was determined as "500" RFU and the second ST as "200" RFU.

As shown in Fig. 11a, when FST of "200" RFU was adopted, the Ct value as a first cross-point ($CP_1$) between the FST and the amplification curve was determined as 1.24. On the other hand, when VST was adopted, the Ct value as a first cross-point ($CP_2$) over VST was determined as 32.02.

As shown in Figs. 11b and 11c, the cut-off value for distinguishing the presence or absence of target nucleic acids was set as Ct<40. When FST of "500" RFU was adopted, the results of $10^{-6}$ diluted RNA showed Ct 43.41 which represents inaccurate amounts of a target nucleic acid.

Furthermore, as the cut-off value for distinguishing the presence or absence of target nucleic acids was set as Ct<40, the Ct 43.41 means the absence of target nucleic acid which corresponds to false negative results. When VST was adopted, the results of $10^{-6}$ diluted RNA showed Ct 39.82 which represents accurate amounts of target nucleic acids and positive results.

Therefore, it would be appreciated that the VST method establishing variable signal thresholds differentially can eliminate errors of misinterpreting a point of generating an initial noise signal in initial amplification cycles as Ct value. Furthermore, the VST method is capable of determining more accurately Ct value, thereby eliminating errors in determination of target nucleic acid concentration.

As described above, the present invention can detect and quantify the target nucleic acid in more reliable and accurate manner by determining accurate Ct value through the setting of the appropriate signal threshold.

**Claims**

1. A method for analyzing a sample, comprising:

   (a) obtaining a value of signal at each of cycles of a signal-generating process using the sample to provide values of signals at the cycles;
   (b) applying a threshold value to each of the cycles such that a plurality of threshold values are applied to the cycles; wherein the threshold values of at least two cycles among the cycles are different from each other;
   (c) identifying one or more cycles satisfying a threshold criterion determined by each of the threshold values; and
   (d) analyzing the sample by using the identified cycle or cycles in the step (c).

2. The method according to claim 1, wherein the analyzing the sample is to determine qualitatively or quantitatively the presence or absence of an analyte in the sample.

3. The method according to claim 1, wherein the signal-generating process is a process to provide an amplification curve.

4. The method according to claim 1, wherein the values of signals are values of signals generated from the signal-generating process or mathematically modified values of the signals generated from the signal-generating process.

5. The method according to claim 1, wherein the threshold values are determined in such a manner that with respect to a threshold-changed cycle (TCC), a function formed by a set of pre-TCC cycles and threshold values to be applied to the pre-TCC cycles is different from a function formed by a set of post-TCC cycles and threshold values to be applied to the post-TCC cycles.

6. The method according to claim 1, wherein the threshold criterion is to have a value of signal the same as or more than the threshold value.

7. The method according to claim 1, wherein the step (b) further comprises applying an additional threshold value to at least one cycle among the cycles.

8. The method according to claim 3, wherein the analyzing the sample is to determine the presence of a target nucleic acid molecule in the sample and the identifying one or more cycles satisfying the threshold criterion is to determine $C_t$ value of the amplification curve.

9. The method according to claim 3, wherein the analyzing the sample is to determine the presence of a target nucleic acid molecule in the sample and the identifying one or more cycles satisfying the threshold criterion is to determine an end-point cycle of a baseline region of the amplification curve.

10. A method for correcting a raw data set of an amplification reaction using a signal-generating means, comprising:

    (a) obtaining the raw data set containing (i) amplification cycles of the amplification reaction and (ii) values of signals obtained from the signal-generating means at the amplification cycles;
    (b) determining a baseline region by determining both a start-point cycle and an end-point cycle of the baseline region using the raw data set;
    (c) establishing a function for a best-fit line of the baseline region using at least two data points of the raw data set within the baseline region; and
    (d) obtaining a corrected data set by subtracting values of the function for the best-fit line from the values of the signals of the raw data set; wherein the corrected data set contains (i) the amplification cycles of the amplification reaction and (ii) the resultants of the subtraction; wherein in step (b) the end-point cycle of the baseline region is determined by a process comprising:

    (b1) applying a baseline threshold value to each of the amplification cycles such that a plurality of baseline threshold values are applied to the cycles; wherein the baseline threshold values of at least two cycles among the cycles are different from each other;
    (b2) identifying one or more cycles satisfying a baseline threshold criterion determined by each of the baseline threshold values; and
    (b3) determining the end-point cycle of the baseline region by using the identified cycle or cycles in the step (b2).

11. The method according to claim 10, wherein the step (a) further comprises plotting the raw data set to provide an amplification curve and the step (d) further comprises plotting the corrected data set to provide a corrected amplification curve.

12. The method according to claim 10, wherein the baseline threshold values for the amplification cycles are determined in such a manner that with respect to a baseline threshold-changed cycle (BTCC), a first function formed by a set of pre-BTCC cycles and baseline threshold values to be applied to the pre-BTCC cycles is different from a second function formed by a set of post-BTCC cycles and baseline threshold values to be applied to the post-BTCC cycles.

13. The method according to claim 10, wherein the amplification cycles are classified into at least two different groups in terms of a baseline threshold-changed cycle (BTCC); wherein cycles classified into a group is continuous, and cycles classified into a group have the same baseline threshold value, and cycles classified into immediately adjacent-different groups have different baseline threshold values from each other.

14. The method according to claim 10, wherein the identification in the step (b2) is performed by comparing a slope calculated for each of the amplification cycles using the raw data set with a baseline threshold value for each of the amplification cycles.

15. The method according to claim 14, wherein the slope is a slope calculated by a least square method using a data point of a certain cycle and at least one data point of a cycle or cycles before and/or after the certain cycle.

16. The method according to claim 10, wherein in step (b) the end-point cycle of the baseline region is determined with a cycle not less than a minimum baseline end-point cycle (MBEC).

17. The method according to claim 16, wherein the end-point cycle of the baseline region is determined by a process comprising:

(i) obtaining a slope calculated for each of the amplification cycles;
(ii) comparing the slope with the baseline threshold value for each amplification cycle to obtain a candidate of the end-point cycle of the baseline region; and
(ii) comparing the candidate of the end-point cycle with the MBEC, wherein when the candidate of the end-point cycle is more than the MBEC, the candidate is determined as the end-point cycle.

18. The method according to claim 10, wherein the method further comprises applying an additional baseline threshold value to at least one cycle among the cycles.

19. The method according to claim 10, wherein establishing the function for the best-fit line of the baseline region is performed by a linear regression analysis using at least two data points within the baseline region.

20. A computer readable storage medium containing instructions to configure a processor to perform a method for analyzing a sample, the method comprising:

(a) receiving a value of signal at each of cycles of a signal-generating process using the sample to provide values of signals at the cycles;
(b) applying a threshold value to each of the cycles such that a plurality of threshold values are applied to the cycles; wherein the threshold values of at least two cycles among the cycles are different from each other;
(c) identifying one or more cycles satisfying a threshold criterion determined by each of the threshold values; and
(d) analyzing the sample by using the identified cycle or cycles in the step (c).

21. A computer readable storage medium containing instructions to configure a processor to perform a method for correcting a raw data set of an amplification reaction using a signal-generating means, the method comprising:

(a) receiving the raw data set containing (i) amplification cycles of the amplification reaction and (ii) values of signals obtained from the signal-generating means at the amplification cycles;
(b) determining a baseline region by determining both a start-point cycle and an end-point cycle of the baseline region using the raw data set;
(c) establishing a function for a best-fit line of the baseline region using at least two data points of the raw data set within the baseline region; and
(d) obtaining a corrected data set by subtracting values of the function for the best-fit line from the values of the signals of the raw data set; wherein the corrected data set contains (i) the amplification cycles of the amplification reaction and (ii) the resultants of the subtraction; wherein in step (b) the end-point cycle of the baseline region is determined by a process comprising:

(b1) applying a baseline threshold value to each of the amplification cycles such that a plurality of baseline threshold values are applied to the cycles; wherein the baseline threshold values of at least two cycles among the cycles are different from each other;
(b2) identifying one or more cycles satisfying a baseline threshold criterion determined by each of the baseline threshold values; and
(b3) determining the end-point cycle of the baseline region by using the identified cycle or cycles in the step (b2).

22. A device for analyzing a sample, comprising (a) a computer processor and (b) the computer readable storage medium of claim 20 coupled to the computer processor.

23. A device for correcting a raw data set of an amplification reaction using a signal-generating means, comprising (a) a computer processor and (b) the computer readable storage medium of claim 21 coupled to the computer processor.

24. A computer program to be stored on a computer readable storage medium to configure a processor to perform a method for analyzing a sample, the method comprising:

(a) receiving a value of signal at each of cycles of a signal-generating process using the sample to provide values of signals at the cycles;
(b) applying a threshold value to each of the cycles such that a plurality of threshold values are applied to the cycles; wherein the threshold values of at least two cycles among the cycles are different from each other;
(c) identifying one or more cycles satisfying a threshold criterion determined by each of the threshold values; and
(d) analyzing the sample by using the identified cycle or cycles in the step (c).

25. A computer program to be stored on a computer readable storage medium to configure a processor to perform a method for correcting a raw data set of an amplification reaction using a signal-generating means, the method comprising:

(a) receiving the raw data set containing (i) amplification cycles of the amplification reaction and (ii) values of signals obtained from the signal-generating means at the amplification cycles;
(b) determining a baseline region by determining both a start-point cycle and an end-point cycle of the baseline region using the raw data set;
(c) establishing a function for a best-fit line of the baseline region using at least two data points of the raw data set within the baseline region; and
(d) obtaining a corrected data set by subtracting values of the function for the best-fit line from the values of the signals of the raw data set; wherein the corrected data set contains (i) the amplification cycles of the amplification reaction and (ii) the resultants of the subtraction; wherein in step (b) the end-point cycle of the baseline region is determined by a process comprising:

(b1) applying a baseline threshold value to each of the amplification cycles such that a plurality of baseline threshold values are applied to the cycles; wherein the baseline threshold values of at least two cycles among the cycles are different from each other;
(b2) identifying one or more cycles satisfying a baseline threshold criterion determined by each of the baseline threshold values; and
(b3) determining the end-point cycle of the baseline region by using the identified cycle or cycles in the step (b2).

**Patentansprüche**

1. Verfahren zum Analysieren einer Probe, umfassend:

(a) Erhalten eines Signalwertes bei jedem der Zyklen eines Signalerzeugungsvorgangs unter Verwendung der Probe, um Signalwerte bei den Zyklen bereitzustellen;
(b) Anwenden eines Schwellenwertes auf jeden der Zyklen, sodass eine Vielzahl von Schwellenwerten auf die Zyklen angewendet wird; wobei die Schwellenwerte von mindestens zwei der Zyklen sich voneinander unterscheiden;
(c) Ermitteln eines oder mehrerer der Zyklen, der/die ein Schwellenwertkriterium erfüllt/erfüllen, welches durch jeden der Schwellenwerte bestimmt wird; und
(d) Analysieren der Probe unter Verwendung des/der in Schritt (c) ermittelten Zyklus/Zyklen.

2. Verfahren nach Anspruch 1, wobei die Analyse der Probe darin besteht, qualitativ oder quantitativ das Vorhandensein oder Nichtvorhandensein eines Analyts in der Probe zu bestimmen.

3. Verfahren nach Anspruch 1, wobei der Signalerzeugungsvorgang ein Prozess zum Bereitstellen einer Verstärkungskurve ist.

4. Verfahren nach Anspruch 1, wobei die Signalwerte durch den Signalerzeugungsvorgang erzeugt werden oder mathematisch veränderte Werte der durch den Signalerzeugungsvorgang erzeugten Signale sind.

**5.** Verfahren nach Anspruch 1, wobei die Schwellenwerte derart bestimmt werden, dass hinsichtlich eines Zyklus mit verändertem Schwellenwert (Engl. *threshold-changed cycle;* TCC) eine durch eine Reihe von Vor-TCC-Zyklen und auf die Vor-TCC-Zyklen anzuwendende Schwellenwerte gebildete Funktion sich von einer durch eine Reihe von Nach-TCC-Zyklen und auf die Nach-TCC-Zyklen anzuwendende Schwellenwerte gebildeten Funktion unterscheidet.

**6.** Verfahren nach Anspruch 1, wobei das Schwellenwertkriterium darin besteht, einen Signalwert zu haben, welcher identisch mit oder größer als der Schwellenwert ist.

**7.** Verfahren nach Anspruch 1, wobei Schritt (b) ferner das Anwenden eines zusätzlichen Schwellenwertes auf mindestens einen der Zyklen umfasst.

**8.** Verfahren nach Anspruch 3, wobei die Analyse der Probe darin besteht, das Vorhandensein eines Zielnukleinsäuremoleküls in der Probe zu bestimmen, und die Ermittlung von einem oder mehreren das Schwellenwertkriterium erfüllende Zyklen darin besteht, den $C_t$-Wert der Verstärkungskurve zu bestimmen.

**9.** Verfahren nach Anspruch 3, wobei die Analyse der Probe darin besteht, das Vorhandensein eines Zielnukleinsäuremoleküls in der Probe zu bestimmen, und die Ermittlung von einem oder mehreren das Schwellenwertkriterium erfüllende Zyklen darin besteht, einen Endpunktzyklus eines Basislinien-Bereiches der Verstärkungskurve zu bestimmen.

**10.** Verfahren zum Korrigieren eines Rohdatensatzes einer Verstärkungsreaktion unter Verwendung einer Signalerzeugungseinrichtung, umfassend:

(a) Erhalten des Rohdatensatzes, der Folgendes umfasst: (i) Verstärkungszyklen der Verstärkungsreaktion und (ii) Signalwerte, die durch die Signalerzeugungseinrichtung bei den Verstärkungszyklen erhalten wurden;
(b) Bestimmen eines Basislinien-Bereiches durch Bestimmen eines Ausgangspunktzyklus sowie eines Endpunktzyklus des Basislinien-Bereiches unter Verwendung des Rohdatensatzes;
(c) Festlegen einer Funktion für eine Ausgleichsgerade (Engl. *best-fit line*) des Basislinien-Bereiches unter Verwendung mindestens zweier Datenpunkte des Rohdatensatzes innerhalb des Basislinien-Bereiches; und
(d) Erhalten eines korrigierten Datensatzes durch Abziehen von Werten der Funktion für die Ausgleichsgerade von den Werten der Signale des Rohdatensatzes; wobei der korrigierte Datensatz Folgendes umfasst: (i) die Verstärkungszyklen der Verstärkungsreaktion und (ii) die Resultanten der Subtraktion; wobei in Schritt (b) der Endpunktzyklus des Basislinien-Bereiches durch einen Vorgang bestimmt wird, welcher Folgendes umfasst:

(b1) Anwenden eines Basislinien-Schwellenwertes auf jeden der Verstärkungszyklen, sodass eine Vielzahl von Basislinien-Schwellenwerten auf die Zyklen angewendet wird; wobei die Basislinien-Schwellenwerte von mindestens zwei der Zyklen sich voneinander unterscheiden;
(b2) Ermitteln eines oder mehrerer der Zyklen, der/die ein Basislinien-Schwellenwertkriterium erfüllt/erfüllen, welches durch jeden der Basislinien-Schwellenwerte bestimmt wird; und
(b3) Bestimmen des Endpunktzyklus des Basislinien-Bereiches unter Verwendung des/der in Schritt (b2) ermittelten Zyklus/Zyklen.

**11.** Verfahren nach Anspruch 10, wobei Schritt (a) ferner das Plotten des Rohdatensatzes umfasst, um eine Verstärkungskurve bereitzustellen, und wobei Schritt (d) ferner das Plotten des korrigierten Datensatzes umfasst, um eine korrigierte Verstärkungskurve bereitzustellen.

**12.** Verfahren nach Anspruch 10, wobei die Basislinien-Schwellenwerte für die Verstärkungszyklen derart bestimmt werden, dass hinsichtlich eines Zyklus mit verändertem Basislinien-Schwellenwert (Engl. *baseline threshold-changed cycle,* BTCC) eine erste, durch eine Reihe von Vor-BTCC-Zyklen und auf die Vor-BTCC-Zyklen anzuwendende Basislinien-Schwellenwerte gebildete Funktion sich von einer zweiten Funktion unterscheidet, welche durch eine Reihe von Nach-BTCC-Zyklen und auf die Nach-BTCC-Zyklen anzuwendende Basislinien-Schwellenwerte gebildet wird.

**13.** Verfahren nach Anspruch 10, wobei die Verstärkungszyklen in mindestens zwei verschiedene Gruppen bezüglich eines Zyklus mit verändertem Basislinien-Schwellenwert (Engl. *baseline threshold-changed cycle*; BTCC) eingeordnet werden; wobei in eine Gruppe eingeordnete Zyklen stetig sind und in eine Gruppe eingeordnete Zyklen denselben Basislinien-Schwellenwert aufweisen und wobei in direkt aneinander angrenzende, unterschiedliche Gruppen eingeordnete Zyklen sich voneinander unterscheidende Basislinien-Schwellenwerte aufweisen.

14. Verfahren nach Anspruch 10, wobei die Ermittlung in Schritt (b2) durch Vergleichen einer Steigung durchgeführt wird, welche für jeden der Verstärkungszyklen unter Verwendung des Rohdatensatzes mit einem Schwellenwert für jeden der Verstärkungszyklen berechnet wird.

15. Verfahren nach Anspruch 14, wobei die Steigung durch eine Methode der kleinsten Fehlerquadrate unter Verwendung eines Datenpunktes eines bestimmten Zyklus und mindestens eines Datenpunktes eines Zyklus oder von Zyklen vor und/oder nach dem bestimmten Zyklus berechnet wird.

16. Verfahren nach Anspruch 10, wobei in Schritt (b) der Endpunktzyklus des Basislinien-Bereiches mit einem Zyklus bestimmt wird, welcher nicht unter einem minimalen Basislinien-Endpunktzyklus (Engl. *minimum baseline end-point cycle; MBEC*) liegt.

17. Verfahren nach Anspruch 16, wobei der Endpunktzyklus des Basislinien-Bereiches durch einen Vorgang bestimmt wird, welcher Folgendes umfasst:

(i) Erhalten einer für jeden der Verstärkungszyklen berechneten Steigung;
(ii) Vergleichen der Steigung mit dem Basislinien-Schwellenwert für jeden Verstärkungszyklus, um einen Kandidaten des Endpunktzyklus des Basislinien-Bereiches zu erhalten; und
(ii) Vergleichen des Kandidaten des Endpunktzyklus mit dem MBEC, wobei der Kandidat als Endpunktzyklus bestimmt wird, wenn der Kandidat des Endpunktzyklus größer als der MBEC ist.

18. Verfahren nach Anspruch 10, wobei das Verfahren ferner das Anwenden eines zusätzlichen Basislinien-Schwellenwertes auf mindestens einen der Zyklen umfasst.

19. Verfahren nach Anspruch 10, wobei das Festlegen der Funktion für die Ausgleichsgerade des Basislinien-Bereiches durch eine lineare Regressionsanalyse unter Verwendung mindestens zweier Datenpunkte innerhalb des Basislinien-Bereiches durchgeführt wird.

20. Computerlesbares Speichermedium, das Anweisungen enthält, um einen Prozessor so zu konfigurieren, dass dieser ein Verfahren zum Analysieren einer Probe durchführt, wobei das Verfahren Folgendes umfasst:

(a) Empfangen eines Signalwertes bei jedem der Zyklen eines Signalerzeugungsvorgangs unter Verwendung der Probe, um Signalwerte bei den Zyklen bereitzustellen;
(b) Anwenden eines Schwellenwertes auf jeden der Zyklen, sodass eine Vielzahl von Schwellenwerten auf die Zyklen angewendet wird; wobei die Schwellenwerte von mindestens zwei der Zyklen sich voneinander unterscheiden;
(c) Ermitteln eines oder mehrerer der Zyklen, der/die ein Schwellenwertkriterium erfüllt/erfüllen, welches durch jeden der Schwellenwerte bestimmt wird; und
(d) Analysieren der Probe unter Verwendung des/der in Schritt (c) ermittelten Zyklus/Zyklen.

21. Computerlesbares Speichermedium, das Anweisungen enthält, um einen Prozessor so zu konfigurieren, dass dieser ein Verfahren zum Korrigieren eines Rohdatensatzes einer Verstärkungsreaktion unter Verwendung einer Signalerzeugungseinrichtung durchführt, wobei das Verfahren Folgendes umfasst:

(a) Empfangen des Rohdatensatzes, der Folgendes umfasst: (i) Verstärkungszyklen der Verstärkungsreaktion und (ii) Signalwerte, die durch die Signalerzeugungseinrichtung bei den Verstärkungszyklen erhalten wurden;
(b) Bestimmen eines Basislinien-Bereiches durch Bestimmen eines Ausgangspunktzyklus sowie eines Endpunktzyklus des Basislinien-Bereiches unter Verwendung des Rohdatensatzes;
(c) Festlegen einer Funktion für eine Ausgleichsgerade des Basislinien-Bereiches unter Verwendung mindestens zweier Datenpunkte des Rohdatensatzes innerhalb des Basislinien-Bereiches; und
(d) Erhalten eines korrigierten Datensatzes durch Abziehen von Werten der Funktion für die Ausgleichsgerade von den Werten der Signale des Rohdatensatzes; wobei der korrigierte Datensatz Folgendes umfasst: (i) die Verstärkungszyklen der Verstärkungsreaktion und (ii) die Resultanten der Subtraktion; wobei in Schritt (b) der Endpunktzyklus des Basislinien-Bereiches durch einen Vorgang bestimmt wird, welcher Folgendes umfasst:

(b1) Anwenden eines Basislinien-Schwellenwertes auf jeden der Verstärkungszyklen, sodass eine Vielzahl von Basislinien-Schwellenwerten auf die Zyklen angewendet wird, wobei die Basislinien-Schwellenwerte von mindestens zwei der Zyklen sich voneinander unterscheiden;
(b2) Ermitteln eines oder mehrerer der Zyklen, der/die ein Basislinien-Schwellenwertkriterium erfüllt/erfüllen, welches durch jeden der Basislinien-Schwellenwerte bestimmt wird; und
(b3) Bestimmen des Endpunktzyklus des Basislinien-Bereiches unter Verwendung des/der in Schritt (b2) ermittelten Zyklus/Zyklen.

22. Vorrichtung zum Analysieren einer Probe, umfassend (a) einen Computerprozessor und (b) das computerlesbare Speichermedium nach Anspruch 20, welches an den Computerprozessor gekoppelt ist.

23. Vorrichtung zum Korrigieren eines Rohdatensatzes einer Verstärkungsreaktion unter Verwendung einer Signalerzeugungseinrichtung, umfassend (a) einen Computerprozessor und (b) das computerlesbare Speichermedium nach Anspruch 21, welches an den Computerprozessor gekoppelt ist.

24. Computerprogramm, das auf einem computerlesbaren Speichermedium zum Konfigurieren eines Prozessors zum Durchführen eines Verfahrens zum Analysieren einer Probe gespeichert wird, wobei das Verfahren Folgendes umfasst:

(a) Empfangen eines Signalwertes bei jedem der Zyklen eines Signalerzeugungsvorgangs unter Verwendung der Probe, um Signalwerte bei den Zyklen bereitzustellen;
(b) Anwenden eines Schwellenwertes auf jeden der Zyklen, sodass eine Vielzahl von Schwellenwerten auf die Zyklen angewendet wird; wobei die Schwellenwerte von mindestens zwei der Zyklen sich voneinander unterscheiden;
(c) Ermitteln eines oder mehrerer der Zyklen, der/die ein Schwellenwertkriterium erfüllt/erfüllen, welches durch jeden der Schwellenwerte bestimmt wird; und
(d) Analysieren der Probe unter Verwendung des/der in Schritt (c) ermittelten Zyklus/Zyklen.

25. Computerprogramm, das auf einem computerlesbaren Speichermedium zum Konfigurieren eines Prozessors zum Durchführen eines Verfahrens zum Korrigieren eines Rohdatensatzes einer Verstärkungsreaktion unter Verwendung einer Signalerzeugungseinrichtung gespeichert wird, wobei das Verfahren Folgendes umfasst:

(a) Empfangen des Rohdatensatzes, der Folgendes umfasst: (i) Verstärkungszyklen der Verstärkungsreaktion und (ii) Signalwerte, die durch die Signalerzeugungseinrichtung bei den Verstärkungszyklen erhalten wurden;
(b) Bestimmen eines Basislinien-Bereiches durch Bestimmen eines Ausgangspunktzyklus sowie eines Endpunktzyklus des Basislinien-Bereiches unter Verwendung des Rohdatensatzes;
(c) Festlegen einer Funktion für eine Ausgleichsgerade des Basislinien-Bereiches unter Verwendung mindestens zweier Datenpunkte des Rohdatensatzes innerhalb des Basislinien-Bereiches; und
(d) Erhalten eines korrigierten Datensatzes durch Abziehen von Werten der Funktion für die Ausgleichsgerade von den Werten der Signale des Rohdatensatzes; wobei der korrigierte Datensatz Folgendes umfasst: (i) die Verstärkungszyklen der Verstärkungsreaktion und (ii) die Resultanten der Subtraktion; wobei in Schritt (b) der Endpunktzyklus des Basislinien-Bereiches durch einen Vorgang bestimmt wird, welcher Folgendes umfasst:

(b1) Anwenden eines Basislinien-Schwellenwertes auf jeden der Verstärkungszyklen, sodass eine Vielzahl von Basislinien-Schwellenwerten auf die Zyklen angewendet wird, wobei die Basislinien-Schwellenwerte von mindestens zwei der Zyklen sich voneinander unterscheiden;
(b2) Ermitteln eines oder mehrerer der Zyklen, der/die ein Basislinien-Schwellenwertkriterium erfüllt/erfüllen, welches durch jeden der Basislinien-Schwellenwerte bestimmt wird; und
(b3) Bestimmen des Endpunktzyklus des Basislinien-Bereiches unter Verwendung des/der in Schritt (b2) ermittelten Zyklus/Zyklen.

**Revendications**

1. Procédé d'analyse d'un échantillon, comprenant :

(a) obtenir une valeur de signal à chacun des cycles d'un processus de génération de signaux en utilisant l'échantillon pour fournir des valeurs de signaux aux cycles ;
(b) appliquer une valeur de seuil à chacun des cycles de sorte qu'une pluralité de valeurs de seuil est appliquée aux cycles ; dans lequel les valeurs de seuil d'au moins deux cycles parmi les cycles sont différentes les unes des autres ;

(c) identifier un ou plusieurs cycles qui répond(ent) à un critère de seuil déterminé par chacune des valeurs de seuil ; et

(d) analyser l'échantillon en utilisant le cycle ou les cycles identifié(s) dans l'étape (c).

2. Procédé selon la revendication 1, dans lequel l'analyse de l'échantillon consiste en déterminer la présence ou la non-existence d'un analyte dans l'échantillon de manière qualitative ou quantitative.

3. Procédé selon la revendication 1, dans lequel le processus de génération de signaux est un processus pour fournir une courbe d'amplification.

4. Procédé selon la revendication 1, dans lequel les valeurs de signaux sont des valeurs de signaux générées par le processus de génération de signaux ou des valeurs modifiées mathématiquement des signaux générés par le processus de génération de signaux.

5. Procédé selon la revendication 1, dans lequel les valeurs de seuil sont déterminées de telle manière que, par rapport à un cycle avec un seuil changé (en anglais *threshold-changed cycle* ; TCC), une fonction formée par un jeu de cycles pré-TCC et des valeurs de seuil à appliquer aux cycles pré-TCC est différente d'une fonction formée par un jeu de cycles post-TCC et des valeurs de seuil à appliquer aux cycles post-TCC.

6. Procédé selon la revendication 1, dans lequel le critère de seuil consiste en avoir une valeur de signal qui est identique ou supérieure à la valeur de seuil.

7. Procédé selon la revendication 1, dans lequel l'étape (b) en outre comprend l'application d'une valeur de seuil additionnelle à au moins un cycle parmi les cycles.

8. Procédé selon la revendication 3, dans lequel l'analyse de l'échantillon consiste en déterminer la présence d'une molécule d'acide nucléique cible dans l'échantillon, et l'identification d'un ou de plusieurs cycles qui répond(ent) au critère de seuil consiste en déterminer la valeur $C_t$ de la courbe d'amplification.

9. Procédé selon la revendication 3, dans lequel l'analyse de l'échantillon consiste en déterminer la présence d'une molécule d'acide nucléique cible dans l'échantillon, et l'identification d'un ou de plusieurs cycles qui répond(ent) au critère de seuil consiste en déterminer un cycle de point terminal d'une région de niveau de référence de la courbe d'amplification.

10. Procédé de correction d'un ensemble de données brutes d'une réaction d'amplification en utilisant un moyen de génération de signaux, comprenant :

(a) obtenir l'ensemble de données brutes comportant : (i) des cycles d'amplification de la réaction d'amplification et (ii) des valeurs de signaux obtenues par le moyen de génération de signaux aux cycles d'amplification ;

(b) déterminer une région de niveau de référence en déterminant un cycle de point de départ ainsi qu'un cycle de point terminal de la région de niveau de référence en utilisant l'ensemble de données brutes ;

(c) élaborer une fonction pour une courbe d'ajustement (en anglais *best-fit line*) de la région de niveau de référence en utilisant au moins deux points de données de l'ensemble de données brutes dans la région de niveau de référence ; et

(d) obtenir un ensemble de données corrigé en soustrayant des valeurs de la fonction pour la courbe d'ajustement des valeurs des signaux de l'ensemble de données brutes ; dans lequel l'ensemble de données corrigé comporte (i) les cycles d'amplification de la réaction d'amplification et (ii) les résultants de la soustraction ; dans lequel, dans l'étape (b), le cycle de point terminal de la région de niveau de référence est déterminé par un processus comprenant :

(b1) appliquer une valeur de seuil de niveau de référence à chacun des cycles d'amplification de sorte qu'une pluralité de valeurs de seuil de niveau de référence est appliqué aux cycles ; dans lequel les valeurs de seuil de niveau de référence d'au moins deux cycles parmi les cycles sont différentes les unes des autres ;

(b2) identifier un ou plusieurs cycles qui répond(ent) à un critère de seuil de niveau de référence déterminé par chacune des valeurs de seuil de niveau de référence ; et

(b3) déterminer le cycle de point terminal de la région de niveau de référence en utilisant le cycle ou les cycles identifié(s) dans l'étape (b2).

11. Procédé selon la revendication 10, dans lequel l'étape (a) en outre comprend tracer l'ensemble de données brutes pour fournir une courbe d'amplification et dans lequel l'étape (d) en outre comprend tracer l'ensemble de données corrigé pour fournir une courbe d'amplification corrigée.

12. Procédé selon la revendication 10, dans lequel les valeurs de seuil de niveau de référence pour les cycles d'amplification sont déterminées de telle manière que, par rapport à un cycle avec un seuil de niveau de référence changé (en anglais *baseline threshold-changed cycle* ; BTCC), une première fonction formée par un jeu de cycles pré-BTCC et des valeurs de seuil de niveau de référence à appliquer aux cycles pré-BTCC est différente d'une deuxième fonction formée par un jeu de cycles post-BTCC et des valeurs de seuil de niveau de référence à appliquer aux cycles post-BTCC.

13. Procédé selon la revendication 10, dans lequel les cycles d'amplification sont classés dans au moins deux groupes différents en termes d'un cycle avec un seuil de niveau de référence changé (en anglais *baseline threshold-changed cycle* ; BTCC) ; dans lequel des cycles classés dans un groupe sont continus, et des cycles classés dans un groupe ont la même valeur de seuil de niveau de référence, et des cycles classés dans des groupes différents immédiatement adjacents ont des valeurs de seuil de niveau de référence différentes.

14. Procédé selon la revendication 10, dans lequel l'identification dans l'étape (b2) est exécutée en comparant une pente calculée pour chacun des cycles d'amplification en utilisant l'ensemble de données brutes avec une valeur de seuil de niveau de référence pour chacun des cycles d'amplification.

15. Procédé selon la revendication 14, dans lequel la pente est une pente calculée par une méthode des moindres carrés en utilisant un point de données d'un cycle particulier et au moins un point de données d'un cycle ou de cycles avant et/ou après le cycle particulier.

16. Procédé selon la revendication 10, dans lequel dans l'étape (b), le cycle de point terminal de la région de niveau de référence est déterminé avec un cycle au moins égal à un cycle de point terminal de niveau de référence minimal (en anglais *minimum baseline end-point cycle ; MBEC*).

17. Procédé selon la revendication 16, dans lequel le cycle de point terminal de la région de niveau de référence est déterminé par un processus comprenant :

(i) obtenir une pente calculée pour chacun des cycles d'amplification ;
(ii) comparer la pente avec la valeur de seuil de niveau de référence pour chaque cycle d'amplification pour obtenir un candidat du cycle de point terminal de la région de niveau de référence ; et
(ii) comparer le candidat du cycle de point terminal avec le MBEC, dans lequel, quand le candidat du cycle de point terminal est supérieur au MBEC, le candidat est déterminé comme le cycle de point terminal.

18. Procédé selon la revendication 10, dans lequel le procédé en outre comprend l'application d'une valeur de seuil de niveau de référence additionnelle à au moins un cycle parmi les cycles.

19. Procédé selon la revendication 10, dans lequel l'élaboration de la fonction pour la courbe d'ajustement de la région de niveau de référence est exécutée par une analyse de régression linéaire en utilisant au moins deux points de données dans la région de niveau de référence.

20. Support de stockage lisible par ordinateur, contenant des instructions pour configurer un processeur pour exécuter un procédé d'analyse d'un échantillon, ledit procédé comprenant :

(a) recevoir une valeur de signal à chacun des cycles d'un processus de génération de signaux en utilisant l'échantillon pour fournir des valeurs de signaux aux cycles ;
(b) appliquer une valeur de seuil à chacun des cycles de sorte qu'une pluralité de valeurs de seuil est appliquée aux cycles ; dans lequel les valeurs de seuil d'au moins deux cycles parmi les cycles sont différentes les unes des autres ;
(c) identifier un ou plusieurs cycles qui répond(ent) à un critère de seuil déterminé par chacune des valeurs de seuil ; et
(d) analyser l'échantillon en utilisant le cycle ou les cycles identifié(s) dans l'étape (c).

**21.** Support de stockage lisible par ordinateur, contenant des instructions pour configurer un processeur pour exécuter un procédé de correction d'un ensemble de données brutes d'une réaction d'amplification en utilisant un moyen de génération de signaux, ledit procédé comprenant :

(a) recevoir l'ensemble de données brutes comportant : (i) des cycles d'amplification de la réaction d'amplification et (ii) des valeurs de signaux obtenues par le moyen de génération de signaux aux cycles d'amplification ;
(b) déterminer une région de niveau de référence en déterminant un cycle de point de départ ainsi qu'un cycle de point terminal de la région de niveau de référence en utilisant l'ensemble de données brutes ;
(c) élaborer une fonction pour une courbe d'ajustement de la région de niveau de référence en utilisant au moins deux points de données de l'ensemble de données brutes dans la région de niveau de référence ; et
(d) obtenir un ensemble de données corrigé en soustrayant des valeurs de la fonction pour la courbe d'ajustement des valeurs des signaux de l'ensemble de données brutes ; dans lequel l'ensemble de données corrigé comporte (i) les cycles d'amplification de la réaction d'amplification et (ii) les résultants de la soustraction ; dans lequel, dans l'étape (b), le cycle de point terminal de la région de niveau de référence est déterminé par un processus comprenant :

(b1) appliquer une valeur de seuil de niveau de référence à chacun des cycles d'amplification de sorte qu'une pluralité de valeurs de seuil de niveau de référence est appliqué aux cycles ; dans lequel les valeurs de seuil de niveau de référence d'au moins deux cycles parmi les cycles sont différentes les unes des autres ;
(b2) identifier un ou plusieurs cycles qui répond(ent) à un critère de seuil de niveau de référence déterminé par chacune des valeurs de seuil de niveau de référence ; et
(b3) déterminer le cycle de point terminal de la région de niveau de référence en utilisant le cycle ou les cycles identifié(s) dans l'étape (b2).

**22.** Dispositif pour analyser un échantillon, comprenant (a) un processeur d'ordinateur et (b) le support de stockage lisible par ordinateur selon la revendication 20 couplé au processeur d'ordinateur.

**23.** Dispositif pour corriger un ensemble de données brutes d'une réaction d'amplification en utilisant un moyen de génération de signaux, comprenant (a) un processeur d'ordinateur et (b) le support de stockage lisible par ordinateur selon la revendication 21 couplé au processeur d'ordinateur.

**24.** Programme d'ordinateur à être enregistré sur un support de stockage lisible par ordinateur pour configurer un processeur pour exécuter un procédé d'analyse d'un échantillon, ledit procédé comprenant :

(a) recevoir une valeur de signal à chacun des cycles d'un processus de génération de signaux en utilisant l'échantillon pour fournir des valeurs de signaux aux cycles ;
(b) appliquer une valeur de seuil à chacun des cycles de sorte qu'une pluralité de valeurs de seuil est appliquée aux cycles ; dans lequel les valeurs de seuil d'au moins deux cycles parmi les cycles sont différentes les unes des autres ;
(c) identifier un ou plusieurs cycles qui répond(ent) à un critère de seuil déterminé par chacune des valeurs de seuil ; et
(d) analyser l'échantillon en utilisant le cycle ou les cycles identifié(s) dans l'étape (c).

**25.** Programme d'ordinateur à être enregistré sur un support de stockage lisible par ordinateur pour configurer un processeur pour exécuter un procédé de correction d'un ensemble de données brutes d'une réaction d'amplification en utilisant un moyen de génération de signaux, ledit procédé comprenant :

(a) recevoir l'ensemble de données brutes comportant : (i) des cycles d'amplification de la réaction d'amplification et (ii) des valeurs de signaux obtenues par le moyen de génération de signaux aux cycles d'amplification ;
(b) déterminer une région de niveau de référence en déterminant un cycle de point de départ ainsi qu'un cycle de point terminal de la région de niveau de référence en utilisant l'ensemble de données brutes ;
(c) élaborer une fonction pour une courbe d'ajustement de la région de niveau de référence en utilisant au moins deux points de données de l'ensemble de données brutes dans la région de niveau de référence ; et
(d) obtenir un ensemble de données corrigé en soustrayant des valeurs de la fonction pour la courbe d'ajustement des valeurs des signaux de l'ensemble de données brutes ; dans lequel l'ensemble de données corrigé comporte (i) les cycles d'amplification de la réaction d'amplification et (ii) les résultants de la soustraction ; dans lequel, dans l'étape (b), le cycle de point terminal de la région de niveau de référence est déterminé par un processus comprenant :

(b1) appliquer une valeur de seuil de niveau de référence à chacun des cycles d'amplification de sorte qu'une pluralité de valeurs de seuil de niveau de référence est appliqué aux cycles ; dans lequel les valeurs de seuil de niveau de référence d'au moins deux cycles parmi les cycles sont différentes les unes des autres ;
(b2) identifier un ou plusieurs cycles qui répond(ent) à un critère de seuil de niveau de référence déterminé par chacune des valeurs de seuil de niveau de référence ; et
(b3) déterminer le cycle de point terminal de la région de niveau de référence en utilisant le cycle ou les cycles identifié(s) dans l'étape (b2).

# Fig. 1A

<u>10</u>

| Obtaining Value of Signal at Each of Cycles | S10 |

↓

| Applying Variable Threshold Values | S20 |

↓

| Identifying One or More Cycles Satisfying Threshold Criterion | S30 |

↓

| Analyzing Sample | S40 |

# Fig. 1B

<u>100</u>

| Obtaining Raw Data Set | S110 |

| Determining Baseline Region by slope curve and baseline threshold | S120 |

| Establishing Function for Best-fit Line | S130 |

| Obtaining Corrected Data Set | S140 |

# Fig. 2

# Fig. 3

# Fig. 4A

# Fig. 4B

# Fig. 5

# Fig. 6

# Fig. 7

Concentration of FluA[3]

1) CP represents a first cross-point between the slope curve and the baseline threshold.

2) BT was used to determine the end-point cycle of a baseline region.

3) Template is a genomic RNA of human influenza A virus (Flu A).

# Fig. 8A

## High conc.

[1] MBEC represents minimum baseline end-point cycle.

[2] $CP_1$ represents a first cross-point between the slope curve and Baseline Threshold when the MBEC is not applied. $CP_2$ represents a first cross-point between the slope curve and the Baseline threshold when the MBEC is applied.

[3] $B_1$ represents a baseline region from the start-point cycle (S) to the end-point cycle ($E_1$) when the MBEC is not applied. $B_2$ represents a baseline region from the start-point cycle (S) to the end-point cycle ($E_2$) when the MBEC is applied.

# Fig. 8B

Low conc.

1) MBEC represents minimum baseline end-point cycle.

2) $CP_1$ represents a first cross-point between the slope curve and the Baseline Threshold when the MBEC is not applied. $CP_2$ represents a first cross-point between the slope curve and the Baseline Threshold when the MBEC is applied.

3) $B_1$ represents a baseline region from the start-point cycle (S) to the end-point cycle ($E_1$) when the MBEC is not applied. $B_2$ represents a baseline region from the start-point cycle (S) to the end-point cycle ($E_2$) when the MBEC is applied.

# Fig. 8C

## Concentration of FluA[4]

High conc.       Low conc.

Slope Curve (BT[2] 30)

Corrected Curve (No MBEC)

Corrected Curve (MBEC)

[1] CP represents a first cross-point between the slope curve and the BT (Baseline Threshold) when the MBEC is applied.

[2] BT was used to determine the end-point cycle of a baseline region.

[3] MBEC represents minimum baseline end-point cycle.

[4] Template is a genomic RNA of human influenza A virus (Flu A).

# Fig. 9

(a)

(b)

(c)

(d)

(e)

(f)

(g)

(h)

# Fig. 10A

High conc.

1) BTCC represents baseline threshold-changed cycle.

2) 1st BT represents baseline threshold(BT) at cycles before the BTCC. 2nd BT represents a baseline threshold at cycles after the BTCC.

3) $CP_1$ represents a first cross-point between the slope curve and the fixed baseline threshold when the VBT is not applied. $CP_2$ represents a first cross-point between the slope curve and the 1st BT and 2nd BT when the VBT is applied.

4) $B_1$ represents a baseline region from the start-point cycle (S) to the end-point cycle ($E_1$) when the VBT is not applied. $B_2$ represents a baseline region from the start-point cycle (S) to the end-point cycle ($E_2$) when the VBT is applied.

# Fig. 10B

Low conc.

1) BTCC represents baseline threshold-changed cycle.

2) 1st BT represents baseline threshold(BT) at cycles before the BTCC. 2nd BT represents baseline threshold at cycles after the BTCC.

3) $CP_1$ represents a first cross-point between the slope curve and the fixed baseline threshold when the VBT is not applied. $CP_2$ represents a first cross-point between the slope curve and the 1st BT and 2nd BT when the VBT is applied.

4) $B_1$ represents a baseline region from the start-point cycle (S) to the end-point cycle ($E_1$) when the VBT is not applied. $B_2$ represents a baseline region from the start-point cycle (S) to the end-point cycle ($E_2$) when the VBT is applied.

# Fig. 10C

## Concentration of FluA[4]

**High conc.**

**Low conc.**

Slope Curve

Corrected Curve (FBT[2] 30 and No VBT)

Corrected Curve (VBT)

[1] CP represents a first cross-point between the slope curve and the baseline threshold.

[2] FBT represents fixed baseline threshold.

[3] BTCC represents baseline threshold-changed cycle.

[4] Template is a genomic RNA of human influenza A virus (Flu A).

# Fig. 11A

| Analysis | Signal threshold | Ct value |
|---|---|---|
| FST[2] Method | 200 | 1.24 |
| VST[3] Method | 500 / 200 | 32.02 |

[1] RFU represents relative fluorescence units.

[2] FST represents Fixed Signal Threshold.

[3] VST represents Variable Signal Threshold.

[4] STCC represents signal threshold-changed cycle.

[5] 1st ST represents the ST(signal threshold) at the cycles before the STCC, and 2nd ST represents the ST at the cycles after the STCC.

[6] $CP_1$ represents the first cross-point between RFU curve and FST, and $CP_2$ represents the first cross-point between RFU curve and VST.

# Fig. 11B

1) RFU represents relative fluorescence units.
2) FST represents Fixed Signal Threshold.
3) VST represents Variable Signal Threshold.
4) STCC represents signal threshold-changed cycle.
5) Templates are serially diluted genomic RNAs of human influenza A virus (Flu A).

# Fig. 11C

| Diluted RNA[4] | Ct value | | | Result of Flu A | |
|---|---|---|---|---|---|
| | FST[2] 500 | VST[3] 500/200 | Cut-off Value[1] | FST 500 | VST 500/200 |
| $10^{-3}$ | 33.56 | 33.56 | | Positive | Positive |
| $10^{-4}$ | 37.01 | 37.01 | | Positive | Positive |
| $10^{-5}$ | 39.93 | 38.21 | < 40.00 | Positive | Positive |
| $10^{-6}$ | 43.41 | 39.82 | | Negative | Positive |
| $10^{-7}$ | N/A | N/A | | Negative | Negative |

[1] Cut-off value allows distinguishing the presence or absence of Flu A.
[2] FST represents Fixed Signal Threshold.
[3] VST represents Variable Signal Threshold.
[4] Templates are serially diluted genomic RNAs of human influenza A virus (Flu A).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6516276 B
- US 6228593 B
- US 7349809 B
- US 7115229 B
- US 6816790 B
- US 4683195 A, Mullis
- US 4683202 A
- US 4800159 A
- US 8219324 B
- US 2012231458 A
- WO 2011130184 A
- EP 1804172 A
- EP 1801237 A
- US 5210015 A
- US 6117635 A
- US 7537886 B
- US 6977295 B
- US 7348141 B
- US 5876930 A
- WO 2012096523 A
- WO 2013115442 A
- KR 2013012312 W
- WO 2011037306 A
- US 6358691 B
- US 6194149 B
- WO 9001069 A
- EP 439182 A
- WO 9300447 A
- US 5556751 A
- EP 497272 A
- US 5888779 A
- KR 1020140132229
- KR 1020140191924
- KR 1020150051080

### Non-patent literature cited in the description

- **SAIKI et al.** *Science,* 1985, vol. 230, 1350-1354
- **LOGAN J et al.** Real-Time PCR: Current Technology and Applications. Caister Academic Press, 2009
- **TYAGI et al.** *Nature Biotechnology,* 1996, vol. 14 (3), 303
- **WHITCOMBE et al.** *Nature Biotechnology,* 1999, vol. 17, 804-807
- **NAZARENKO et al.** *Nucleic Acids Research,* 1997, vol. 25 (12), 2516-2521
- **DUCK P et al.** *Biotechniques,* 1990, vol. 9, 142-148
- **SHERRILL CB et al.** *Journal of the American Chemical Society,* 2004, vol. 126, 4550-4556
- **D. J. FRENCH et al.** *Molecular and Cellular Probes,* 2001, vol. 13, 363-374
- **BERNARD PS et al.** *Clin Chem,* 2000, vol. 46, 147-148
- **WIEDMANN M et al.** Ligase chain reaction (LCR)- overview and applications. *PCR Methods and Applications,* February 1994, vol. 3 (4), 51-64
- **CAHILL P et al.** *Clin Chem.,* 1991, vol. 37 (9), 1482-5
- **G T WALKER et al.** *Nucleic Acids Res.,* 1992, vol. 20 (7), 16911696
- **COMPTON.** *J. Nature,* 1991, vol. 350 (6313), 912
- **HOFMANN WP et al.** *J Clin Virol.,* 2005, vol. 32 (4), 289-93
- **HUTCHISON C.A. et al.** *Proc. Natl Acad. Sci. USA.,* 2005, vol. 102, 1733217336
- **JOSEPH SAMBROOK et al.** Molecular Cloning, A Laboratory Manual. Cold Spring Harbor Laboratory Press, 2001